# EUROPEAN PATENT APPLICATION

(11) **EP 4 513 185 A1**
(43) Date of publication of application: **26.02.2025**
(21) Application number: 23192753.4
(22) Date of filing: 22.08.2023
(51) Int. Cl.: G01N 33/50, A61K 39/00

(54) **EARLY RESPONSE BIOMARKERS FOR ALLERGEN IMMUNOTHERAPY**

(71) Applicant: Luxembourg Institute of Health (LIH), 1445 Strassen (LU)
(72) Inventor: OLLERT, Markus, 5616 Mondorf-les-Bains (LU); HE, Feng, 4276 Esch-Sur Alzette (LU)
(74) Representative: De Clercq & Partners

(57) **Abstract**

The present invention provides an *in vitro* method for predicting responsiveness to an allergen immunotherapy (AIT) in a subject having an allergy comprising measuring interleukin-6 (IL-6) and Suppressor Of Cytokine Signaling 3 (SOCS3), and optionally Sphingosine-1-phosphate receptor 1 (S1PR1) and/or B-cell lymphoma 3 (BCL3) in a biological sample from said subject at a time-point in the period spanning from 3 hours before to 24 hours after the timepoint when the maintenance dose of said immunotherapeutic is at least reached by the cumulative dose of said allergy immunotherapeutic. Also provided herein is a kit comprising means specifically adapted for measuring the quantity or expression levels of IL-6 and SOCS3, and optionally S1PR1 and/or BCL3 in a biological sample from a subject and the use thereof for predicting the response of a subject having an allergy to an AIT.

## Description

### TECHNICAL FIELD

The present invention is situated in the field of novel biomarkers and biomarker combinations in the early determination of an individual's response to allergen immunotherapy and kits for use therein.

### BACKGROUND OF THE INVENTION

Repeated administration of relevant specific allergens, also known as allergen-specific immunotherapy (AIT), to patients with allergy is the only curative option available. The process of AIT mimics the frequent stimulations/exposures of microbial factors and natural allergen exposure in pre-school children with the traditional or rural farm lifestyle that secures the normal maturation process of the immune system, which, however, is most likely delayed by the modern westernized lifestyle in many patients with allergy.

AIT should be performed for at least three years to ensure a maximum immune tolerance-inducing effect (Shamji et al., 2022; Tabar et al., 2011). Therefore, most of the studies have focused on the investigation of specific immune changes that occur after the completion of long-lasting AlT. It has been well documented that AIT induces a decrease in soluble and cellular allergy effectors. This has been reflected by a reduction in IgE levels, activation and degranulation of mast cells and basophils as well as frequencies of allergen-specific Th2 cells and innate lymphoid cells (ILCs). In the meantime, the allergic-response dampening factors or cell types, such as IgE-blocking antibody, allergen-specific IgG4 and the immune regulatory subsets with suppressive functions [i.e., regulatory CD4 T cells (Treg) and B cells (Breg)] are enhanced by AlT.

Nevertheless, since AIT takes at least three years, requiring a long-term compliance of patients and being extremely resource-demanding, there is a high need to identify robust early-window cellular or molecular predictive events that could aid in the decision of initiating and continuing clinically successful AIT.

### SUMMARY

Present inventors have developed a diagnostic tool, that can be used for instance as a companion diagnostic to aid in selecting or excluding patients for an allergen immunotherapy (AIT), such as insect venom AlT. More particularly, the present inventors surprisingly found that particular biomarkers can be used as early indicators of allergen immune response, and more particularly response to AlT. For instance it was found that measuring one or more biomarkers selected from the group consisting of interleukin-6 (IL-6), Suppressor Of Cytokine Signaling 3 (SOCS3), Sphingosine-1-phosphate receptor 1 (S1PR1) and B-cell lymphoma 3 (BCL3) in a biological sample from a subject being subjected to said AIT when the cumulative dose of allergy immunotherapeutic during the up-dosing phase at least reaches the maintenance dose of said allergy immunotherapeutic (e.g. at a timepoint in the period spanning from 3 hours before to 24 hours after the timepoint when the maintenance dose of said allergy immunotherapeutic is at least reached by the cumulative dose of said allergy immunotherapeutic) allows to predict the response of said subject to AlT.

As shown in the experimental section, which illustrates certain representative embodiments of the invention, present inventors compared dynamic immune responses of insect venom allergy patients (VAP) to pollen-allergy patients (PAP), who are known low grade-responders with regard to achieving allergy-cure through pollen AIT, as well as to healthy subjects, immediately following AIT launch and found that serological interleukin-6 (IL-6) as well as Suppressor Of Cytokine Signaling 3 (SOCS3), Sphingosine-1-phosphate receptor 1 (S1PR1) and B-cell lymphoma 3 (BCL3) are transiently upregulated in CD4 Th2 cells at 8 hours post AIT launch (i.e. at the timepoint when the cumulative dose of allergy immunotherapeutic during the up-dosing phase of the insect-venom AIT at least reached the maintenance dose of the allergy immunotherapeutic) in VAP compared to PAP, and hence act as build-up phase-response biomarkers of AlT.

Accordingly, the method of present invention provides biomarkers for response to an AlT. More particularly, it allows to identify those patients that are not likely to respond to an AIT at the very early stages of the therapy. As a result thereof, for such subjects it can be decided to discontinue subjecting them to the time- and resources-demanding AIT, to change the dosage regime for said allergy immunotherapeutic or to administer an alternative therapeutic product (e.g. an alternative allergy immunotherapeutic). Moreover, this implies that such subject also does not have to undergo the potentially life-threatening allergen-response test to verify whether or not the subject responded to the AIT, such as an insect-venom allergen-response test delivered through the sting of a living insect (e.g. of the order of hymenoptera) that is currently being performed according to international clinical guidelines, ideally after 6-18 months of insect-venom AlT. In some extreme cases, present invention could save the lives of some patients, such as patients undergoing insect-venom AIT who are unknowingly non-responsive to said AIT while behaving in the presumption that they are responsive to said AIT, e.g. exposing themselves to situations with a large likelihood of being stung by an insect.

Accordingly, a first aspect provides an *in vitro* method for predicting responsiveness to an allergen immunotherapy (AIT) in a subject having an allergy, wherein said AIT comprises an up-dosing phase during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose, the method comprising measuring one or more, such as at least two, preferably at least the first two, biomarkers selected from the group consisting of IL-6, SOCS3, S1PR1 and BCL3 in a biological sample from said subject when the maintenance dose of said allergy immunotherapeutic is at least reached by the cumulative dose of said allergy immunotherapeutic (e.g. at a timepoint in the period spanning from 3 hours before to 24 hours after the timepoint when the maintenance dose of said allergy immunotherapeutic is at least reached by the cumulative dose of said allergy immunotherapeutic).

In particular embodiments, the method further comprises measuring Janus kinase 1 (JAK1) and/or Signal transducer and activator of transcription 3 (STAT3) in said biological sample from the subject.

In particular embodiments, the biological sample is a whole blood sample.

In particular embodiments, circulating IL-6 is measured in a whole blood sample, preferably in a serum sample or a plasma sample.

In particular embodiments, SOCS3, S1PR1, BCL3, JAK1 and/or STAT3 are measured in CD4 T helper (Tₕ) 2 cells.

In particular embodiments, one or more, such as at least two, preferably at least the first two, biomarkers selected from the group consisting of IL-6, SOCS3, S1PR1 and BCL3 are measured at most 2 hours after the timepoint when the maintenance dose of said allergy immunotherapeutic is at least reached by the cumulative dose of said allergy immunotherapeutic.

In particular embodiments, the method comprises the steps of
(a) measuring the quantity or expression levels of one or more, such as at least two, preferably at least the first two, biomarkers selected from the group consisting of IL-6, SOCS3, S1PR1 and BCL3 in the biological sample from the subject when the maintenance dose of said allergy immunotherapeutic is at least reached by the cumulative dose of said allergy immunotherapeutic;
(b) comparing the quantity or expression levels of said one or more, such as at least two, preferably at least the first two, biomarkers selected from the group consisting of IL-6, SOCS3, S1PR1 and BCL3 as measured in (a) with a reference,
(c) finding a difference or no difference of the quantity or expression levels of said one or more, such as at least two, preferably at least the first two, biomarkers selected from the group consisting of IL-6, SOCS3, S1PR1 and BCL3 as measured in (a) from said reference; and
(d) attributing said finding of a difference or no difference to a particular prediction of responsiveness of the subject to said AlT.

In particular embodiments, said method is a method for determining the responsiveness of a subject allergic to insect venom.

In particular embodiments, the method comprises the steps of
(a) measuring the quantity or expression levels of at least IL-6 and SOCS3 in the biological sample from the subject when the maintenance dose of said allergy immunotherapeutic is at least reached by the cumulative dose of said allergy immunotherapeutic;
(b) comparing the quantity or expression levels of at least IL-6 and SOCS3 as measured in (a) with a reference,
(c) finding a difference or no difference of the quantity or expression levels of at least IL-6 and SOCS3 as measured in (a) from said reference; and
(d) attributing said finding of a difference or no difference to a particular prediction of responsiveness of the subject to said AlT.

In particular embodiments, the quantity or expression level of IL-6 and SOCS3 is measured and compared with a reference to determine predicted responsiveness to an insect-venom AlT.

In further embodiments, the reference is the quantity or expression levels of said one or more, such as at least two, preferably at least the first two, biomarkers selected from the group consisting of IL-6, SOCS3, S1PR1 and BCL3 before, at the start or at most 0.5 hour after the start of the AIT in said subject.

In particular embodiments, an increase in the quantity or expression levels of one or more, such as at least two, preferably at least the first two, biomarkers selected from the group consisting of IL-6, SOCS3, S1PR1 and BCL3 in the biological sample from the subject when the maintenance dose of said immunotherapeutic is at least reached by the cumulative dose of said allergy immunotherapeutic, compared to the reference is indicative of responsiveness of the subject to AlT.

In particular embodiments, the subject is a human subject.

A further aspect provides a kit, in particular a kit for predicting the response of a subject having an allergy to an AIT, the kit comprising means specifically adapted for measuring the quantity or expression levels of one or more, such as at least two, preferably at least the first two, biomarkers selected from the group consisting of IL-6, SOCS3, S1PR1 and BCL3 in a biological sample from a subject.

In particular embodiments, said means comprises one or more binding agents specifically binding to said biomarker or to RNA encoding said biomarker.

A further aspect provides the use of the kit as taught herein for predicting the response of a subject having an allergy to an AIT based on the detection of one or more, such as at least two, preferably at least the first two, biomarkers selected from the group consisting of IL-6, SOCS3, S1PR1 and BCL3 in a biological sample of a subject.

A further aspect provides an allergy immunotherapeutic for use in a method of treating an allergy in a subject, wherein the method comprises predicting responsiveness of said subject to said immunotherapeutic using a method as taught herein and continuing administration of said immunotherapeutic to said subject if the subject is predicted to be responsive to said immunotherapeutic.

These and further aspects and preferred embodiments of the invention are described in the following sections and in the appended claims. The subject-matter of the appended claims is hereby specifically incorporated in this specification.

### BRIEF DESCRIPTION OF DRAWINGS

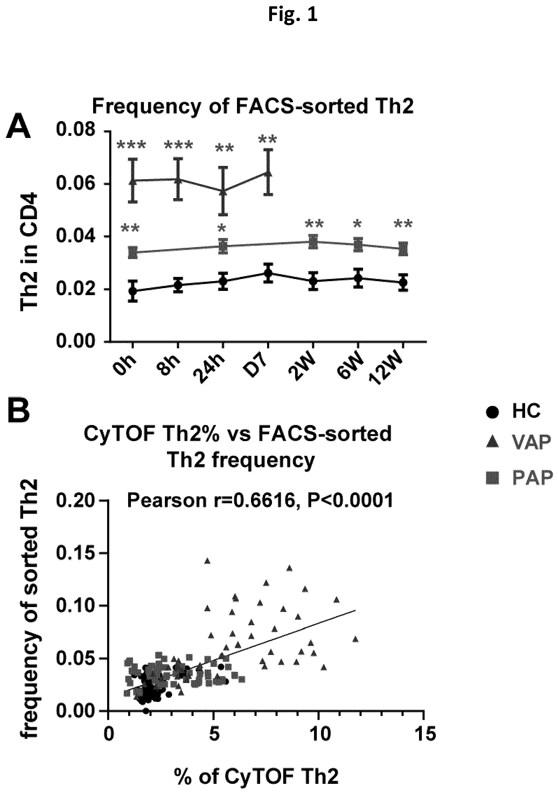

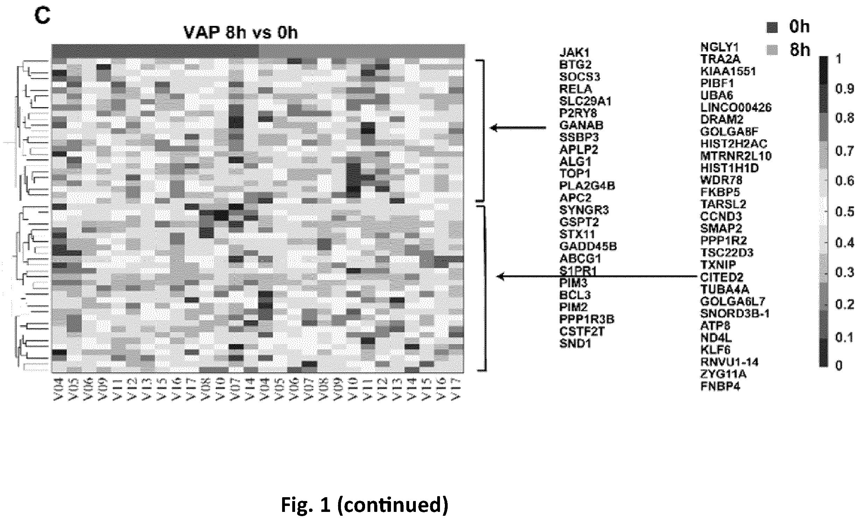

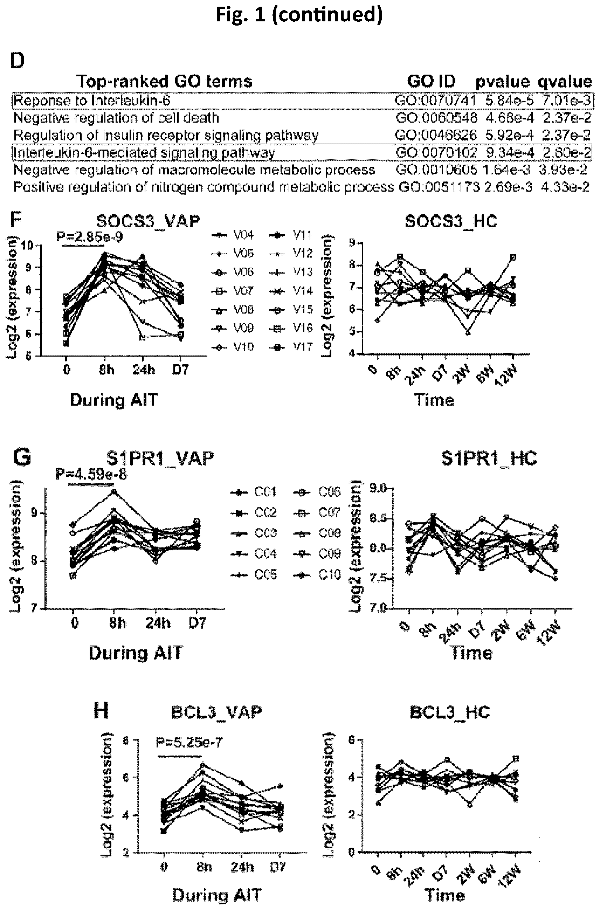

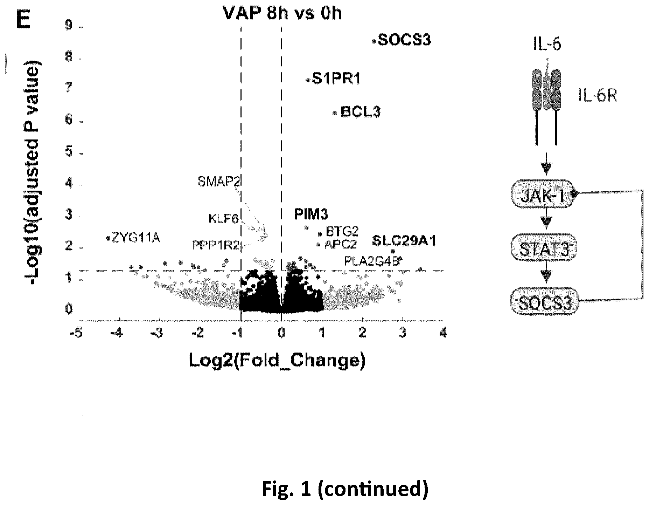

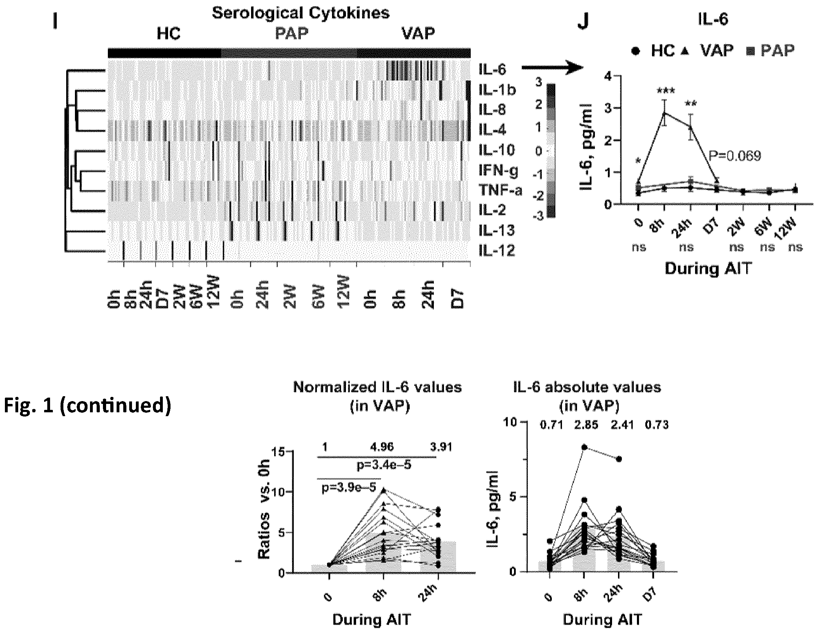

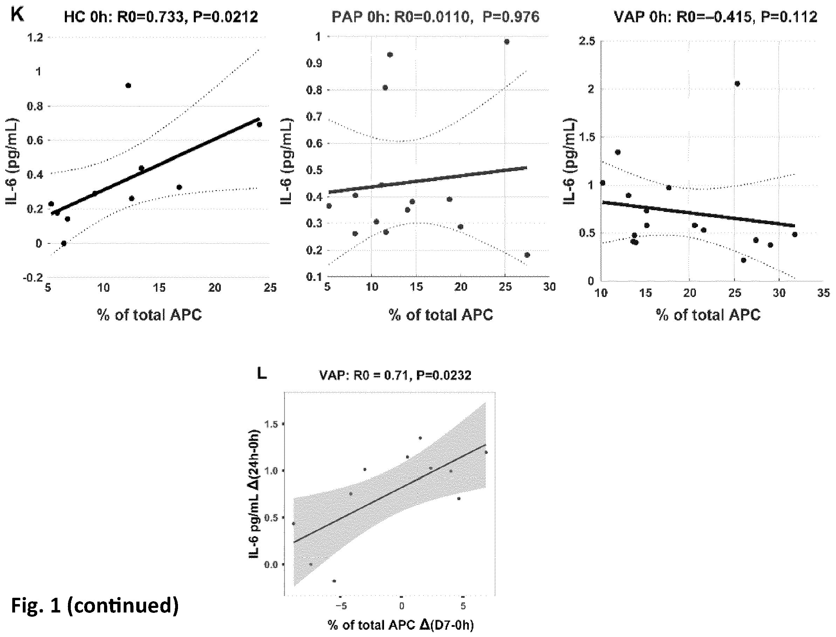

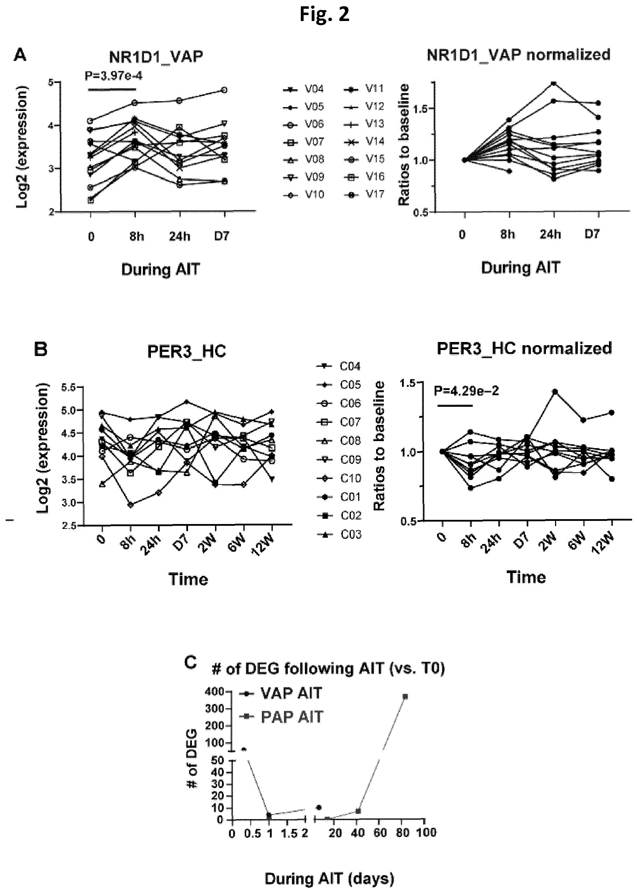

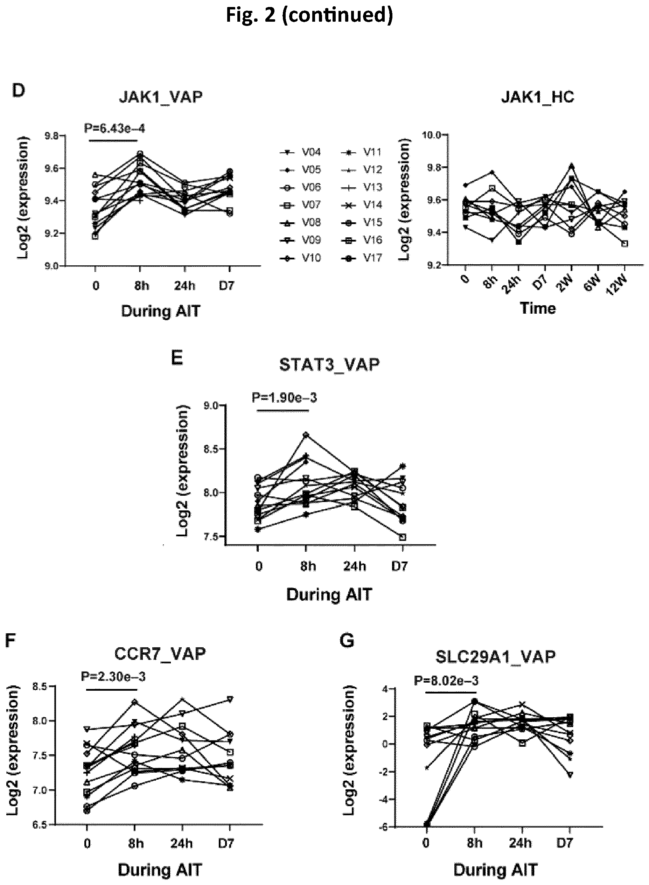

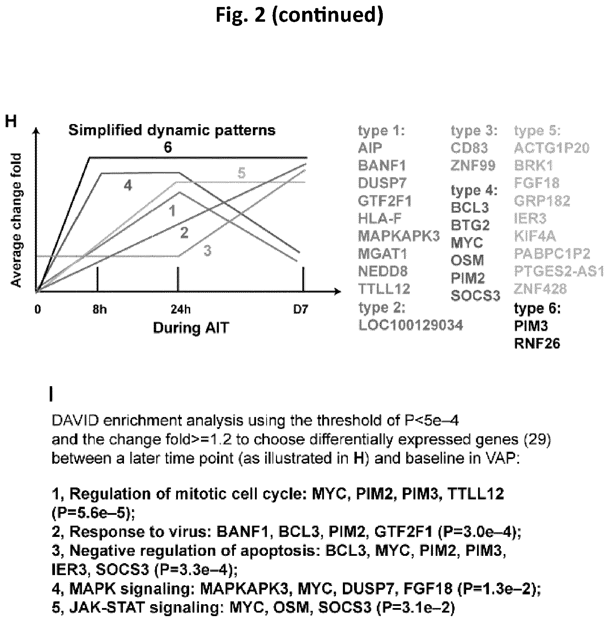

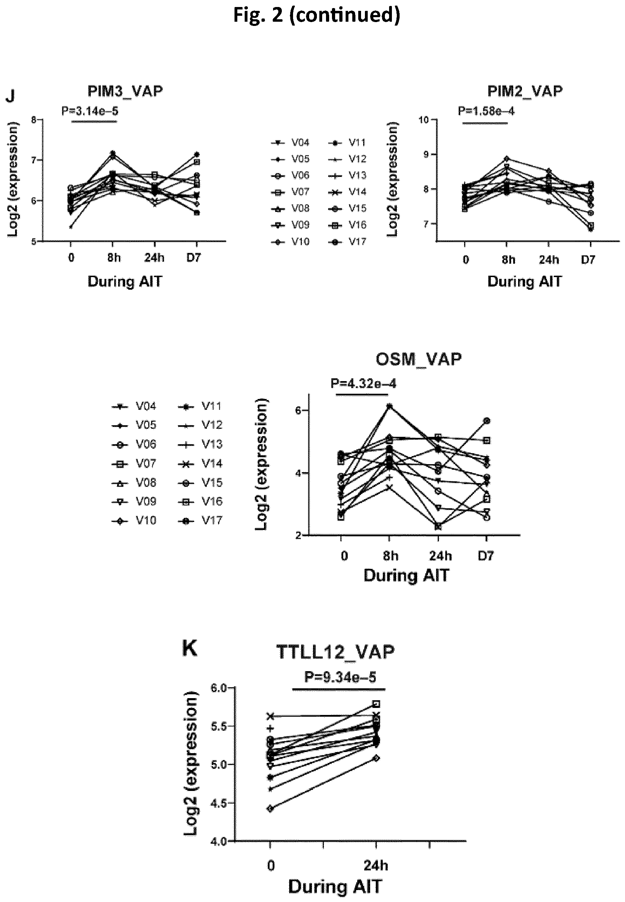

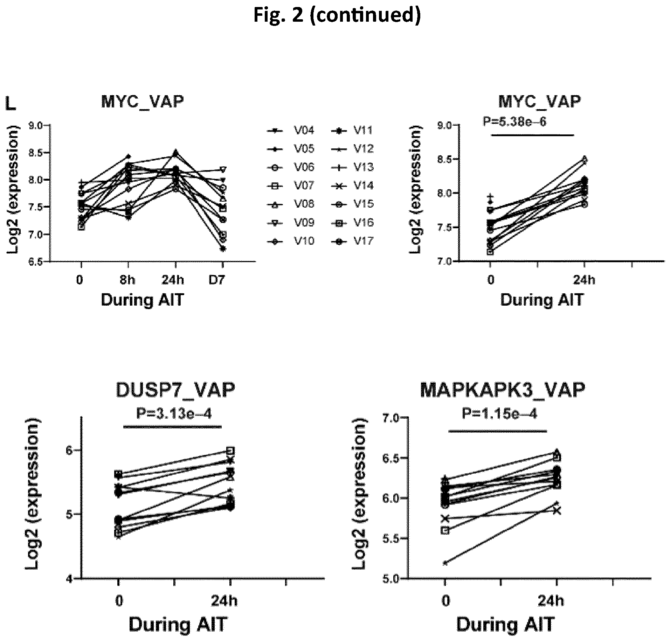

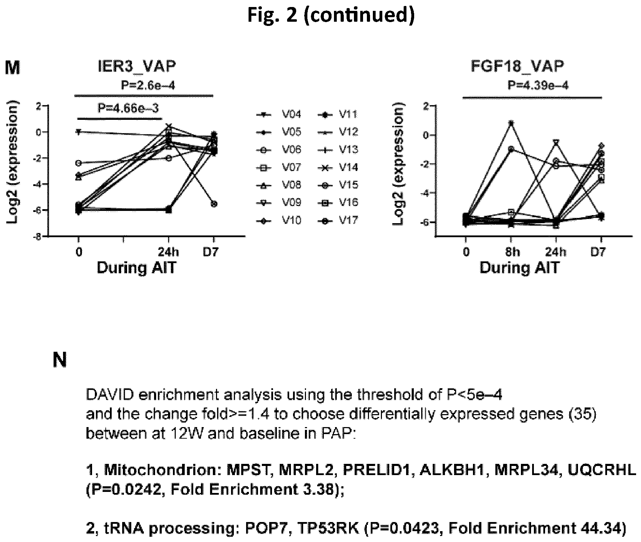

**Fig.1** **Unbiased RNA-seq analysis reveals a pulse of IL-6 signaling in venom-allergy patients (VAP) Th2 cells at 8h following allergen-specific immunotherapy (AIT) launch. A,** Time-course measurement of the frequency of FACS-sorted Th2 cells among living CD4 T cells from fresh blood samples. ***P=5.15e-4 (0h), ***P=8.53e-4 (8h), **P=5.42e-3 (D1), **P=1.61E-3 (D7) between VAP and healthy control (HC); P=**2.70e-3 (0h), P=*0.013 (D1), **P=3.61e-3 (W2), *P=0.0181 (W6) and **P=0.00489 between pollen-allergy patients (PAP) and HC; **B,** Correlation between the frequency of sorted Th2 cells and the percentages of Th2 among total living singlets measured by CyTOF for all the samples of the three groups. **C,** Heatmap of differentially-expressed genes (DEGs) in sorted Th2 cells among VAP at 8h vs. baseline (0h) following AIT launch. **D,** Top-ranked pathways or processes (gene ontology, GO) based on enrichment analysis among those DEGs at 8h vs. 0h in sorted Th2 cells within VAP. **E,** Unbiased volcano plot analysis of DEGs among VAP at 8h vs. 0h in sorted Th2 cells following AIT launch. The selected list of substantially and significantly changed genes were labelled. Right, Schematic of the simplified IL-6 signaling pathway. **F-H,** Dynamic mRNA expression of the gene *SOCS3* (F), *S1PR1* (G) and *BCL3* (H) in sorted Th2 cells following AIT launch among VAP (left) or during the sampling period among HC (right). Displayed P-values were adjusted with FDR. **I,** Heatmap of 10 analyzed serum cytokines among participants of three different groups at various timepoints. **J,** Dynamic serological IL-6 levels, upper panel, dynamic pattern of IL-6 levels over time within three different groups. lower left, IL-6 levels at 0h, 8h and 24h normalized to baseline in VAP following AlT. lower right, Scatter dot plots of absolute IL-6 levels of each VAP during AlT. Mean for each timepoint in both middle and right panels was labelled. **K,** Correlation between serological IL-6 levels and the frequency of total APC among living singlets at baseline in different groups. Spearman correlation coefficient was used. **L,** Correlation between the changed serological IL-6 levels from baseline to 24h and the changed frequency of total APC cells among living singlets from baseline to day 7 within VAP. Data represent mean± standard error of mean (SEM) (A, J); P-value was determined by paired [C, E F-H] or non-paired [A] two-tailed Mann-Whitney test. P-value in J was calculated using paired (middle and right) or non-paired (left) Student t test. ns or unlabeled, not significant, *p<=0.05, **p<=0.01 and ***p<=0.001. AIT, antigen-specific immunotherapy; APC, antigen-presenting cells; FACS, fluorescence-activated cell sorting. HC, healthy controls, n=10; PAP, pollen-allergy patients, n=16; VAP, venom-allergy patients, n=18. See also Figures 2-3.
**Fig. 2****. Extended dynamic Th2-specific RNA-seq analysis. A,** mRNA expression of *NR1D1* (known as REV-ERBa) in sorted Th2 cells from VAP during the first seven days following AIT launch. Right, Expression of *NR1D1* normalized to baseline. **B,** mRNA expression of *PER3* in sorted Th2 cells from HC during 12 weeks of the sampling period. Right, Expression of *PER3* normalized to baseline. **C,** Number of differentially-expressed genes (DEG) in Th2 cells at the given timepoint vs. baseline within the specified patient group following AIT launch. **D,** mRNA expression of *JAK1* in Th2 cells from VAP. Right, mRNA expression of *JAK1* in HC. **E, F, G,** Dynamic mRNA expression of *STAT3* (**E**), *CCR7* (**F**) and *SLC29A1* (**G**) in Th2 cells sorted from VAP following AIT launch. **H,** Simplified upregulation dynamic patterns of mRNA expression. For the genes exhibiting a gradually-increasing pattern, the fold change between peak-time and baseline was at least 1.2 and showed a highly-significant P value (<=5e-4). For the genes exhibiting an increased plateau, the fold change between all timepoints at the plateau and baseline was at least 1.2 and showed a highly-significant P value (<=5e-4) for at least one-time at the plateau. Of note, *S1PR1* was not listed here because it was only peaked at 8h and thus was not counted as one of the genes showing a continuous dynamic pattern. **I, N,** DAVID enrichment analysis (https://david.ncifcrf.gov/tools.jsp, 2021 Update) showing the top-ranked enriched pathways or processes among the genes with a significant change between a later timepoint (as defined in H) and baseline following AIT start in VAP (**I**). The enrichment analysis was also performed for the upregulated genes (criteria were defined in **N**) between W12 and baseline following AIT launch in PAP (**N**). **J**, Dynamic mRNA expression of *PIM3, PIM2* and OSM in Th2 cells from VAP following AIT launch. **K, L,** Uniform upregulation of *TTLL12* (**K**), *MYC, DUSP7* and *MAPKAPK3* (**L**) transcripts in VAP at 24h vs baseline immediately following AIT launch was highlighted. Of note, the expression of *MYC* was also displayed at all the four timepoints during AIT (left). **M,** Dynamic mRNA expression of *IER3* and *FGF18* in Th2 cells within VAP following AIT launch. To avoid diluting the message, the values of IER3 at 8h were not displayed. Each connected line over different timepoints represent one individual; P values in **A, B, D-G, J-M** was determined by paired two-tailed Student T test. HC, healthy controls; PAP, pollen-allergy patients; VAP, venom-allergy patients.

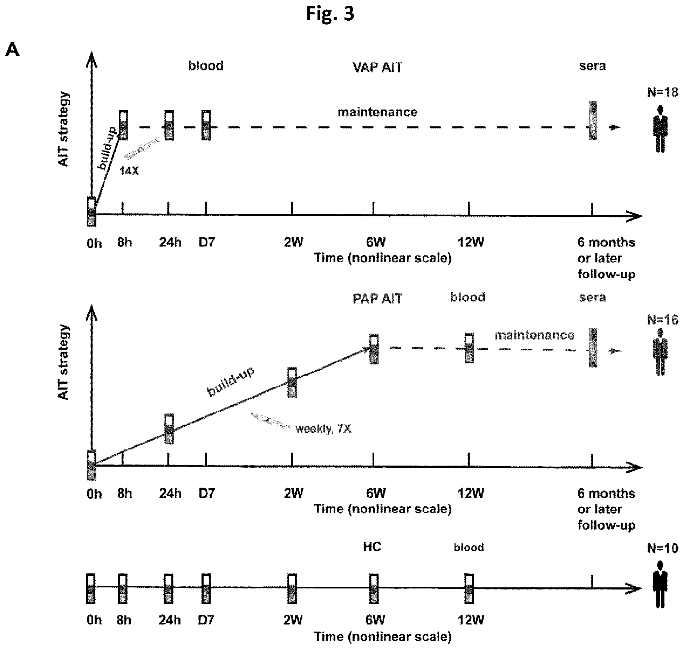

**Fig. 3****. Cohort overview and experimental schematic. A,** Overview of our longitudinal cohort SYSTACT. HC, healthy controls (black), n=10; PAP, pollen-allergy patients (red), n=16; VAP, venom-allergy patients (blue), n=18.

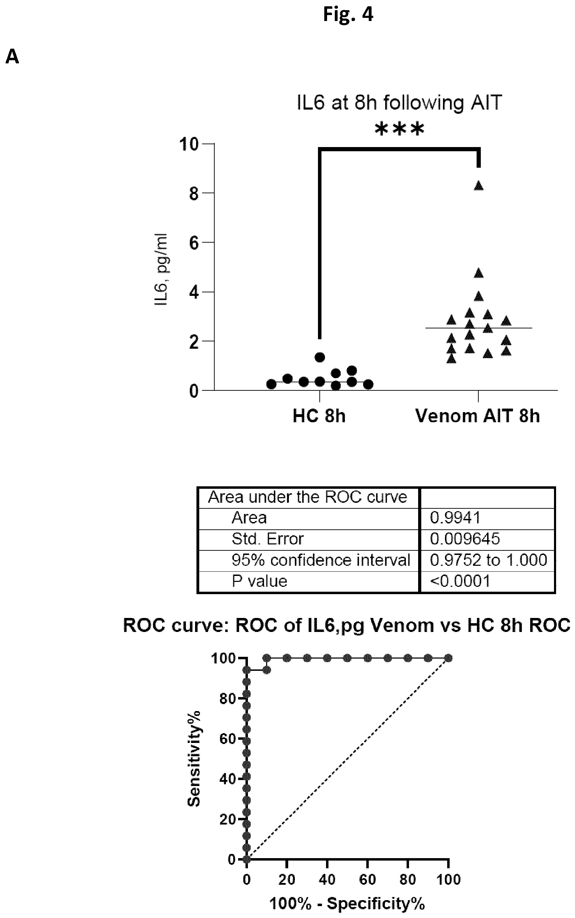

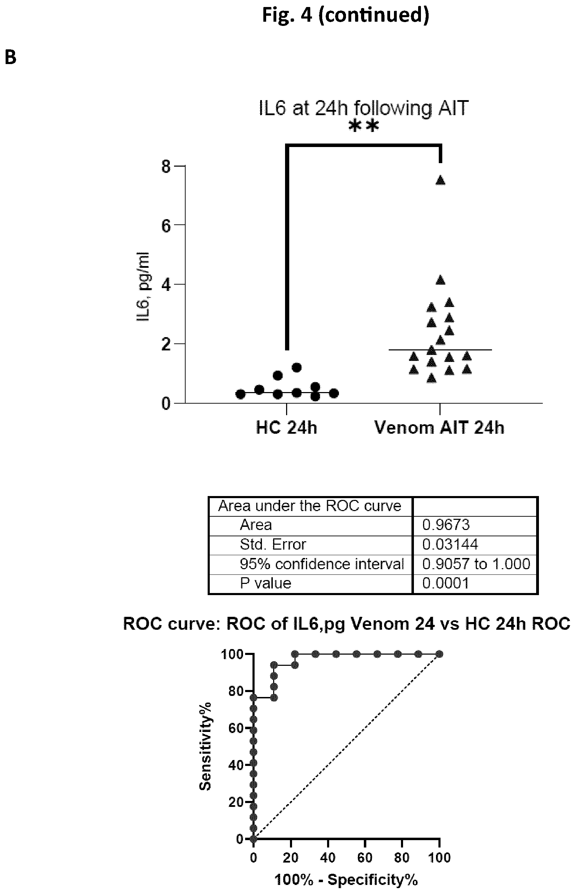

**Fig. 4****. Enhanced serological IL-6 levels could be used as a predictive biomarker of VAP. A,** Receiver operating characteristic (ROC) analysis based on serological IL-6 levels at 8h following AIT launch in VAP vs IL-6 levels in HC without AIT administration. Lower panel shows the group comparison between HC and VAP at 8h. **B,** ROC analysis based on serological IL-6 levels at 24h following AIT launch in VAP vs IL-6 levels in HC without AIT administration. Lower panel shows the group comparison between HC and VAP at 24h. AUC, Area under curve. Each dot represents one individual sample. Median of each group was marked in the group comparison. P-value in the group comparison was determined using an unpaired two-tailed Student t test. P-value from the ROC analysis tests the null hypothesis that the area under the curve really equals 0.50. ns or unlabeled, not significant, *p<=0.05, **p<=0.01 and ***p<=0.001. HC, healthy controls (black), n=10; VAP, venom-allergy patients (blue), n=18.

### DESCRIPTION OF EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation or meaning is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Present inventors found that certain biomarkers are indicative of the response of a subject to an AlT. AIT typically comprises an up-dosing phase (i.e. build-up phase) during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose.

More particularly, present inventors found that responsiveness of a subject to an AIT can be predicted by measuring biomarkers in a sample of said subject shortly after the start of AIT and determining whether there is a difference in quantity or expression level of these markers compared to prior to AIT or compared to typical non-responder levels or poor responder levels of these markers. For example, the inventors have found that measuring one or more biomarkers selected from the group consisting of interleukin-6 (IL-6), Suppressor Of Cytokine Signaling 3 (SOCS3), Sphingosine-1-phosphate receptor 1 (S1PR1) and B-cell lymphoma 3 (BCL3), and preferably at least IL6 and SOCS3, in a biological sample of a subject having an allergy when the maintenance dose of said immunotherapeutic is at least reached by the cumulative dose of said allergy immunotherapeutic, is sufficient to allow the determination of whether said patient is likely to respond to said AlT.

More particularly, as corroborated by the experimental section, which illustrates certain representative embodiments of the invention, present inventors observed a significant and uniform upregulation of serological IL6 secretion at 8 hours (hrs) following the ultra-rush up-dosing AIT launch (i.e. when the total cumulative dose of allergy immunotherapeutic during the up-dosing phase of the insect-venom AIT reached the defined maintenance dose of said allergy immunotherapeutic) in all the patients with insect-venom allergy that responded to AlT. Present inventors did not observe any significant change in the healthy donors or in non-responders or low grade (i.e. poor)-responders (e.g. patients with pollen allergy receiving a subcutaneous injection AIT with pollen extract). In consideration of the highly successful curative rate of insect-venom AIT (more than 90%), the uniform upregulation of serological IL6 at 8hrs relative to baseline in all the patients with insect-venom allergy is a very reliable early-response biomarker. Furthermore, independent of the serological analysis, present inventors also performed a genome-scale unbiased RNA-seq analysis on *ex-vivo* Th2 cells sorted from peripheral blood mononuclear cells (PBMCs). Present inventors found the transcripts of the specific IL6 signalling pathway suppressor, *SOCS3* dramatically and uniformly upregulated among all the venom-insect allergy patients. Present inventors also observed that *S1PR1,* the key receptor guiding the egress of lymphocytes from lymphoid organs into blood has already been significantly and uniformly upregulated in the mRNA level among all the patients with insect-venom allergy. Similar observation has also been made for the gene *BCL3,* a member of non-canonical NFκB pathway.

Accordingly, the present invention provides reliable biomarkers for the early response diagnosis of an AIT in patients with an allergy. In particular embodiments, if when the maintenance dose of said immunotherapeutic is at least reached by the cumulative dose of said allergy immunotherapeutic (e.g. at a timepoint in the period spanning from 3 hours before to 24 hours after the timepoint when the maintenance dose of said allergy immunotherapeutic is at least reached by the cumulative dose of said allergy immunotherapeutic) , upregulation of at least one, preferably at least two, more preferably the first two, biomarkers selected from the group consisting of interleukin-6 (IL-6), Suppressor Of Cytokine Signaling 3 (SOCS3), Sphingosine-1-phosphate receptor 1 (S1PR1) and B-cell lymphoma 3 (BCL3) compared to a reference sample is determined in the given patient, this indicates that the AIT, if continued, will generate a long-term beneficial clinical outcome. In contrast, if when the maintenance dose of said immunotherapeutic is at least reached by the cumulative dose of said allergy immunotherapeutic , upregulation of said at least one, preferably at least two markers, is not determined in the given patient, this indicates that the patient is a non-responder (i.e. is not responsive) to said AIT, and the responsible physicians should consider alternative treatment options for this particular patient.

In view of the above, a first aspect provides an *in vitro* method for predicting responsiveness (or sensitivity or susceptibility) to an allergen immunotherapy (AIT), preferably an insect-venom allergen immunotherapy (AIT), in a subject having an insect-venom allergy, wherein said AIT comprises an up-dosing phase during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose, the method comprising measuring one or more biomarkers selected from the group consisting of IL-6, SOCS3, S1PR1 and BCL3 in a biological sample from said subject at a timepoint in the period spanning from 3 hours before to 24 hours after the timepoint when the maintenance dose of said immunotherapeutic is at least reached by the cumulative dose of said allergy immunotherapeutic . In particular embodiments, the method comprises measuring at least one, preferably at least two, such as at least three or all four of the listed markers. Preferably, the method comprises measuring at least IL-6 and SOCS3.

Also provided is thus the use of one or more biomarkers selected from the group consisting of IL-6, SOCS3, S1PR1 and BCL3 as biomarkers useful for determining responsiveness (or sensitivity or susceptibility) to an AIT, preferably an insect-venom AIT, in a subject having an allergy, preferably an insect-venom allergy. In particular embodiments, at least one, preferably at least two, such as at least three or all four biomarkers are used. Preferably, the method comprises measuring at least IL-6 and SOCS3.

Certain embodiments provide a method as taught herein for indicating an AIT, such as an insect-venom AIT, as a suitable treatment for an allergy, such as an insect-venom allergy, in a subject, the method comprising measuring one or more biomarkers selected from the group consisting of IL-6, SOCS3, S1PR1 and BCL3, preferably at least IL-6 and SOCS3, in a biological sample from said subject after the start of said AIT, such as insect-venom AIT, in said subject and determining the suitability of said AIT treatment based thereon. In particular embodiments, the method comprises measuring at least one, preferably at least two, such as at least three or all four of the listed markers. The biomarker(s) are measured when the maintenance dose of said immunotherapeutic is at least reached by the cumulative dose of said allergy immunotherapeutic, e.g. at a timepoint in the period spanning from 3 hours before to 24 hours after the timepoint when the maintenance dose of said immunotherapeutic is at least reached by the cumulative dose of said allergy immunotherapeutic.

In particular embodiments, if an increase in the quantity or expression levels of at least one, preferably at least two, such as at least three or all four, biomarkers selected from the group consisting of IL-6, SOCS3, S1PR1 and BCL3, preferably at least IL-6 and SOCS3, is observed for the sample compared to a reference sample, the AIT, such as the insect-venom AIT, is indicated as a suitable (effective) treatment, as the subject will clinically benefit from the treatment. In certain embodiments, if no increase in the quantity or expression levels of at least one, preferably at least two, such as at least three or all four, biomarkers selected from the group consisting of IL-6, SOCS3, S1PR1 and BCL3, preferably at least IL-6 and SOCS3, is observed for the sample compared to a reference sample , the AIT, such as the insect-venom AIT, is not indicated as a suitable treatment, as the subject will have no clinical benefit from the treatment.

Certain embodiments provide a method as taught herein for predicting an AIT, such as an insect-venom AIT outcome, in a subject having an allergy, such as an insect-venom allergy, the method comprising measuring the level of at least one, preferably at least two, such as at least three or all four, biomarkers selected from the group consisting of IL-6, SOCS3, S1PR1 and BCL3, preferably at least IL-6 and SOCS3, in a biological sample from said subject after the start of said AIT in said subject and predicting the outcome based on said level. The biomarker(s) are measured when the maintenance dose of said immunotherapeutic is at least reached by the cumulative dose of said allergy immunotherapeutic, e.g. at a timepoint in the period spanning from 3 hours before to 24 hours after the timepoint when the maintenance dose of said immunotherapeutic is at least reached by the cumulative dose of said allergy immunotherapeutic.

The phrases "determining responsiveness" and "predicting responsiveness" may be used interchangeably herein.

The terms "predicting", "prediction" or "predictive" as used herein refers to an advance declaration, indication or foretelling of a response or reaction to a therapy in a subject not (yet) having been treated with the therapy or in a subject having been treated with the therapy but not (yet) for the entire duration of said therapy. For example, a prediction of responsiveness (or sensitivity or susceptibility) to immunotherapy in a subject may indicate that the subject will respond or react to the immunotherapy, for example within a certain time period, e.g., so that the subject will have a clinical benefit from the immunotherapy. A prediction of unresponsiveness (or insensitivity or insusceptibility) to immunotherapy in a subject may indicate that the subject will minimally or not respond or react to the immunotherapy, for example within a certain time period, e.g., so that the subject will have no clinical benefit from the immunotherapy.

The terms "responsiveness", "sensitivity" or "susceptibility" may be used interchangeably herein and refer to the quality that predisposes a subject having an insect-venom allergy to be responsive or reactive to an immunotherapy. A subject is "responsive", "sensitive", or "susceptible" (which terms are used interchangeably) to immunotherapy (i.e., treatment with an immunotherapy agent) if the subject will have a clinical benefit from the treatment.

The terms "unresponsiveness", "insensitivity", "insusceptibility" or "resistance" may be used interchangeably herein and refer to the quality that predisposes a subject having an insect-venom allergy to a minimal (e.g. insignificant) or no response to an immunotherapy. A subject is "unresponsive", "insensitive", "unsusceptible" or "resistant" (which terms are used interchangeably) to an immunotherapy (i.e., treatment with an immunotherapy agent) if the subject will have no clinical benefit from the treatment, e.g. when the therapy is ineffective or fails.

The methods as disclosed herein may allow to make a prediction that a subject having an allergy, such as insect-venom allergy, and starting an allergen immunotherapy, such as insect-venom immunotherapy, will effectively be responsive to said AIT or will be unresponsive thereto. This may in certain embodiments include predicting that a subject having an allergy will have a comparatively low probability (e.g., less than 50%, less than 40%, less than 30%, less than 20% or less than 10%) of being responsive to an allergen immunotherapy; or that a subject having an allergy will have a comparatively high probability (e.g., at least 50%, at least 60%, at least 70%, at least 80% or at least 90%) of being responsive to said allergen immunotherapy.

The methods as disclosed herein may allow to make a prediction that a subject having an allergy, such as insect-venom allergy, and starting an allergen immunotherapy, such as insect-venom immunotherapy, will be a poor responder (e.g. poor clinical response, such as only alleviating some of the allergy symptoms), an intermediate (or moderate) responder or a good responder (e.g. good clinical response) to said AlT.

The term "outcome" generally refers to the evaluation undertaken to assess the results or consequences of management and procedures (i.e., the interventions) used in combatting a disease in order to determine the efficacy, effectiveness, safety, practicability, etc., of these interventions, e.g. in individual cases or series. The term "outcome" as used herein refers to a process of assessing the consequences of treating an individual afflicted with an allergy with allergen immunotherapy, i.e. predicting whether said individual is likely to respond or not to the allergen immunotherapy. The methods as taught herein provide a prediction of how a patient's allergy will progress when treated with an allergen immunotherapy and/or whether there is chance of recovery.

The term "allergen immunotherapy" or "allergen-specific immunotherapy", "AIT", "allergen specific immunotherapy" or "SIT" as used herein refers to a therapy involving contacting a subject repeatedly with an allergy immunotherapeutic, such as by subcutaneous injection, in order to increase tolerance to one or more allergens comprised in said allergy immunotherapeutic. AIT typically consists of build-up (i.e. up-dosing) phase and a maintenance phase to ensure a sustained effect. Most patients maintain their protection from allergic reactions to one or more allergens for many years following allergen immunotherapy. The AIT may be administered in the form of a conventional, a cluster, a rush or an ultra-rush scheme. Allergen immunotherapy is also known in the art as desensitization, hyposensitization, tolerance induction, clinical antigen tolerance induction, peripheral allergen-specific tolerance induction or peripheral antigen-specific tolerance induction. For example, the allergen immunotherapy may be an insect-venom allergen immunotherapy, i.e. an allergy immunotherapy directed at treating an insect-venom allergy. Hence, the term "insect-venom allergen immunotherapy" does not limit the allergen immunotherapy to the use of insect venom as allergen, as, for example, also the immunogenic fragment of allergen(s) may be used in said allergy immunotherapeutic.

In particular embodiments, the subject is suffering from an allergy against an animal and the AIT is based on AIT with an allergen of said animal. In particular embodiments, if the subject is suffering from an insect-venom allergy, the AIT is an insect-venom AlT. In particular embodiments, such as if the subject is suffering from a cat allergy (e.g., allergy to the cat allergen Fel d1), the AIT is cat AIT, preferably Fel d 1-specific AIT with high-dose CpG.

In particular embodiments, the allergen immunotherapy comprises the administration of an allergy immunotherapeutic or allergen immunotherapeutic agent comprising one or more adjuvants.

The term "adjuvant" as used herein refers to any material that increases the humoral or cellular immune response to an antigen. Adjuvants are typically used to accomplish two objectives: slowing down the release of antigens from the injection site, and stimulating the immune system. Non-limiting examples of adjuvants include alum, tyrosine crystals, LP40, poly I:C, Flagellin, microcrystalline tyrosine (MCT), CpG adjuvant, or lipopolysaccharide (LPS) or fragments thereof, such as monophosphoryl A (MPL) adjuvant. The term "insect-venom allergy" used herein refers to an allergic reaction of a subject to an insect venom or a combination of insect venoms, such as upon exposure to an insect venom as a result of an insect bite or sting. Symptoms include skin rashes, itching or hives, shortness of breath, trouble breathing or wheezing, dizziness and/or fainting, stomach pain, vomiting, diarrhea, swelling of the lips, tongue or throat, but are not limited thereto. The symptoms usually occur soon after the person is contacted with the insect venom. In rare cases, they may only occur a few hours later. A person suffering from an insect-venom allergy will produce Immunoglobulin E (IgE) after the first insect sting or bite. If stung again by the same kind of insect, the venom interacts with this specific IgE antibody and causes an allergic reaction leading to the symptoms described above, which are commonly referred to as anaphylaxis. Most patients maintain their protection from allergic reactions to stings for many years following venom immunotherapy.

In particular embodiments, the insect may be of the order *Hymenoptera* (e.g. bees, wasps, ants). For example, an insect of the order *Hymenoptera* may be an insect of the genus bee (Apis), bumblebee (Bombus), wasp (Family: Vespidae, Genus: Dolichovespula, Polistes, Vespula), hornet (Vespa, such as the *Vespa crabro* or *Vespa velutina*) and stinging ant (Family: Formicidae, Genus: Myrmecia, Solenopsis).

In particular embodiments, the insect-venom AIT comprises administering an insect-venom allergy immunotherapeutic or insect-venom allergen immunotherapeutic agent to a subject. In particular embodiments, the insect-venom allergy immunotherapeutic may comprise one or more insect-venom allergens.

The terms "sample" or "biological sample" as used throughout this specification include any biological specimen obtained (isolated, removed) from a subject. Samples may include without limitation whole blood, plasma, serum, whole blood cells, red blood cells, white blood cells (e.g., peripheral blood mononuclear cells), saliva, urine, stool (feces), tears, sweat, sebum, organ tissue, exhalate, nipple aspirate, ductal lavage, tumor exudates, interstitial fluid, synovial fluid, cerebrospinal fluid, lymph, fine needle aspirate, amniotic fluid, any other bodily fluid, exudate or secretory fluid, cell lysates, cellular secretion products, inflammation fluid, semen and vaginal secretions. Preferably, a sample may be readily obtainable by non-invasive or minimally invasive methods, such as blood collection ('liquid biopsy'), urine collection, feces collection, tissue (e.g., tumor tissue) biopsy or fine-needle aspiration, allowing the provision / removal / isolation of the sample from a subject. The term "tissue" as used herein encompasses all types of cells of the body including cells of organs but also including blood and other body fluids recited above. The tissue may be from a living subject or may be cadaveric tissue. In particular embodiments, the sample is a dried blood spot (DBS) or any other capillary blood microsample.

Particularly useful samples are those known to comprise, or expected or predicted to comprise, or known to potentially comprise, or expected or predicted to potentially comprise CD4 T helper (Tₕ) 2 cells.

The person skilled in the art will understand that for the method of present invention, the biological sample is taken from a subject already subjected to said AlT.

In particular embodiments, the biological sample (and/or reference sample) is a whole blood sample.

In particular embodiments, the biological sample (and/or reference sample) comprises, essentially consists of or consists of CD4 T helper (Tₕ) 2 cells, such as CRTH2⁺ CD4 T cells. The person skilled in the art will understand that the CD4 Tₕ2 cells may be defined as CD3+CD4+CD8⁻ non-Treg and CD45RO+CD45RA⁻CD183/CXCR3⁻CD196/CCR6⁻CD194/CCR4+ cells, IL-4 producing CD4 T cells, GATA3⁺ CD4 cells, phospho-STAT6⁺ cells, or any other accepted way to define or identify CD4 Th2 cells or CD4 Th2 cell subsets (for example CRTH2⁺ CD4 T cells). A sample can be obtained from a subject in any way typically used in clinical settings for obtaining a sample comprising the required cells, peptides, proteins, polypeptides or nucleic acid including RNA. If desired, the sample can be mixed with a fluid or purified or amplified or otherwise treated. For examples, samples may be treated in one or more purification steps in order to increase the purity of the desired cells, peptides, proteins, polypeptides or nucleic acid in the sample, or they may be examined without any purification steps. Any peptide, protein, polypeptide or nucleic acid specimen in purified or non-purified form obtained from such sample can be utilized in the methods as taught herein.

In particular embodiments, circulating (i.e. serological) IL-6 is measured in a whole blood sample, preferably in a serum sample or a plasma sample.

In particular embodiments, SOCS3, and optionally one or more of the biomarkers selected from the group consisting of S1PR1, BCL3, JAK1 and STAT3are measured in CD4 T helper (Tₕ) 2 cells.

The biomarkers may be measured in CD4 T helper (Tₕ) 2 cells with or without isolating the cells first from the whole blood sample. Th2 cells do not need to be isolated from the whole blood sample before transcript or mRNA analysis. The measurement of the expression of the biomarkers can be also performed by simultaneously detecting CD4 Th2 cell surface markers and RNA levels of the biomarkers using techniques similar to Prime Flow RNA assay kit (Thermo Fisher Scientific; catalog number 88-18005-210 ), which is an in situ hybridization assay that combines branched-DNA technology with flow cytometry. Alternatively, one could also first immobilize the Th2 cells by coupling the cells to the solid surface of a microarray or macroarray format and simultaneously identify Th2 cells (by detecting relevant protein markers) and measure transcripts within the identified Th2 cells.

In particular embodiments, the CD4 T helper (Tₕ) 2 cells are isolated from said whole blood sample. CD4 T helper (Tₕ) 2 cells may be isolated from a whole blood sample by any method known in the art, such as by first isolating PBMCs from a whole blood sample, subsequently isolating the CD4+ T cells using human CD4 microbeads, CD4 macrobeads or other CD4 magnetic-based enrichment methods, and finally sorting the CD4 T helper (Tₕ) 2 cells using cytometry or microfluidic sorting methods, for example as described in Example 1. In particular embodiments, a whole blood sample is split into two samples, a first whole blood sample for measuring serological IL-6 and a second whole blood sample for isolating CD4 T helper (Tₕ) 2 cells and subsequently measuring SOCS3, and optionally one or more of the biomarkers selected from the group consisting of S1PR1, BCL3, JAK1 and STAT3.

The terms "subject", "individual" or "patient" are used interchangeably throughout this specification, and typically and preferably denote humans, but may also encompass reference to non-human animals, preferably warm-blooded animals, even more preferably mammals, such as, e.g., non-human primates, rodents, canines, felines, equines, ovines, porcines, and the like. The term "non-human animals" includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In certain embodiments, the subject is a non-human mammal. In certain preferred embodiments of the methods or uses as taught herein, the subject is a human subject. In other embodiments, the subject is an experimental animal or animal substitute as a disease model. The term does not denote a particular age or sex. Thus, adult and newborn subjects, as well as fetuses, whether male or female, are intended to be covered. Examples of subjects include humans, dogs, cats, cows, goats, and mice. The term subject is further intended to include transgenic species. In particular embodiments, the subject is a human subject.

Suitable subjects may include without limitation subjects presenting to a physician for the start of an AIT, such as an insect-venom AIT, or subjects undergoing an AIT, such as an insect-venom AIT, like subjects diagnosed with an insect-venom allergy.

The methods as taught herein comprise measuring one or more biomarkers selected from the group consisting of IL-6, SOCS3, S1PR1 and BCL3, preferably at least IL-6 and SOCS3, in a biological sample from said subject. In particular embodiments, IL-6, SOCS3, S1PR1 and BCL3 are human biomarkers, such as human IL-6, human SOCS3, human S1PR1 and human BCL3.

In particular embodiments, IL-6 is an mRNA or protein biomarker. In particular embodiments, SOCS3, S1PR1 and BCL3, are SOCS3 are mRNA biomarkers.

The terms "marker" or "biomarker" are widespread in the art and commonly broadly denote a biological molecule, more particularly an endogenous biological molecule, and/or a detectable portion thereof, whose qualitative and/or quantitative evaluation in a tested object (e.g., in or on a cell, cell population, tissue, organ, or organism, e.g., in a biological sample of a subject) is predictive or informative with respect to one or more aspects of the tested object's phenotype and/or genotype. The terms "marker" and "biomarker" may be used interchangeably throughout this specification.

Table 1 provides the standard nomenclature, as well as the accession numbers for the genomic and mRNA reference sequences of exemplary genes of the present invention and the UniProt entry of the protein encoded by the gene, derived all from Homo sapiens. Source: National Center for Biotechnology Information (NCBI). The entries in Table 1 are presented in the form: gene, name, GenelD, Genbank RefSeq for one or more representative mRNA sequences followed by the Genbank sequence version, and UniProt entry of the protein encoded by the gene.

Exemplary human genes as taught herein may be as annotated under Swissprot/Uniprot (http://www.uniprot.org/) or NCBI Genbank (http://www.ncbi.nlm.nih.gov/) accession numbers given below. A skilled person will appreciate that although only one or more isoforms may be listed below, all isoforms are intended.

**Table 1**

| Gene | Full name | Entrez GenelD | mRNA ID RefSeq | UniProt entry of protein encoded by the gene |
|---|---|---|---|---|
| IL-6 | interleukin-6 | 3569 | NM_000600.5 (transcript variant 1) | P05231.1 |
| SOCS3 | Suppressor Of Cytokine Signaling 3 | 9021 | NM_003955.5 (transcript variant 1) | 014543.1 |
| S1PR1 | Sphingosine-1-phosphate receptor 1 | 1901 | NM_001400.5 (transcript variant 1) | P21453.2 |
| BCL3 | B-cell lymphoma 3 | 602 | NM_005178.5 | P20749.2 |
| JAK1 | Janus kinase 1 | 3716 | NM_002227.4 (transcript variant 1) | P23458.2 |
| STAT3 | Signal transducer and activator of transcription 3 | 6774 | NM_139276.3 (transcript variant 1) | P40763.2 |
| PIM3 | Pim-3 proto-oncogene, serine/threonine kinase | 415116 | NM_001001852.4 | Q86V86.3 |
| SLC29A1 | solute carrier family 29 member 1 (Augustine blood group) | 2030 | NM_001078177.2 (transcript variant 1) | Q99808.3 |
| MYC | MYC proto-oncogene, bHLH transcription factor | 4609 | NM_002467.6 (transcript variant 1) | P01106.2 |
| CCR7 | C-C motif chemokine receptor 7 | 1236 | NM_001838.4 (transcript variant 1) | P32248.2 |
| PIM2 | Pim-2 proto-oncogene, serine/threonine kinase | 11040 | NM_006875.4 | Q9P1W9.1 |
| OSM | oncostatin M | 5008 | NM_020530.6 | P13725.2 |
| TTLL12 | tubulin tyrosine ligase like 12 | 23170 | NM_015140.4 | Q14166.2 |
| DUSP7 | dual specificity phosphatase 7 | 1849 | NM_001947.4 | Q16829.4 |
| MAPKAPK3 | MAPK activated protein kinase 3 | 7867 | NM_001243926.2 (transcript variant 1) | Q16644.1 |

The reference herein to any marker, including any nucleic acid, also encompasses fragments thereof. Hence, the reference herein to measuring (or measuring the quantity of) any one marker may encompass measuring the marker and/or measuring one or more fragments thereof.

For example, any marker and/or one or more fragments thereof may be measured collectively, such that the measured quantity corresponds to the sum amounts of the collectively measured species. In another example, any marker and/or one or more fragments thereof may be measured each individually.

The term "fragment" with reference to a nucleic acid (polynucleotide) generally denotes a 5'- and/or 3'-truncated form of a nucleic acid. Preferably, a fragment may comprise at least about 30%, e.g., at least about 50% or at least about 70%, preferably at least about 80%, e.g., at least about 85%, more preferably at least about 90%, and yet more preferably at least about 95% or even about 99% of the nucleic acid sequence length of said nucleic acid. For example, insofar not exceeding the length of the full-length nucleic acid, a fragment may include a sequence of ≥ 5 consecutive nucleotides, or ≥ 10 consecutive nucleotides, or ≥ 20 consecutive nucleotides, or ≥ 30 consecutive nucleotides, e.g., ≥40 consecutive nucleotides, such as for example ≥ 50 consecutive nucleotides, e.g., ≥ 60, ≥70, ≥80, ≥90, ≥ 100, ≥ 200, ≥300, ≥400, ≥ 500 or ≥ 600 consecutive nucleotides of the corresponding full-length nucleic acid.

The terms encompass fragments arising by any mechanism, in vivo and/or in vitro, such as, without limitation, by alternative transcription or translation, exo- and/or endo-proteolysis, exo- and/or endo-nucleolysis, or degradation of the peptide, polypeptide, protein, or nucleic acid, such as, for example, by physical, chemical and/or enzymatic proteolysis or nucleolysis.

The term "fragment" with reference to a peptide, polypeptide, or protein generally denotes a N- and/or C-terminally truncated form of the peptide, polypeptide, or protein. Preferably, a fragment may comprise at least about 30%, e.g., at least about 50% or at least about 70%, preferably at least about 80%, e.g., at least about 85%, more preferably at least about 90%, and yet more preferably at least about 95% or even about 99% of the amino acid sequence length of said peptide, polypeptide, or protein. For example, insofar not exceeding the length of the full-length peptide, polypeptide, or protein, a fragment may include a sequence of ≥ 5 consecutive amino acids, or ≥ 10 consecutive amino acids, or ≥20 consecutive amino acids, or ≥ 30 consecutive amino acids, e.g., ≥40 consecutive amino acids, such as for example ≥ 50 consecutive amino acids, e.g., ≥ 60, ≥70, ≥80, ≥ 90, ≥100, ≥ 200, ≥ 300 or ≥ 400 consecutive amino acids of the corresponding full-length peptide, polypeptide, or protein.

For example, the at least one, preferably at least two, such as at least three or all four, biomarkers selected from the group consisting of IL-6, SOCS3, S1PR1 and BCL3, more preferably at least IL-6 and SOCS3, may be measured by measuring a peptide fragment of the full-length protein.

The reference herein to any protein, polypeptide or peptide may also encompass variants thereof. The term "variant" of a protein, polypeptide or peptide refers to proteins, polypeptides or peptides the sequence (i.e., amino acid sequence) of which is substantially identical (i.e., largely but not wholly identical) to the sequence of said recited protein or polypeptide, e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical. Preferably, a variant may display such degrees of identity to a recited protein, polypeptide or peptide when the whole sequence of the recited protein, polypeptide or peptide is queried in the sequence alignment (i.e., overall sequence identity).

Sequence identity may be determined using suitable algorithms for performing sequence alignments and determination of sequence identity as know *per se.* Exemplary but non-limiting algorithms include those based on the Basic Local Alignment Search Tool (BLAST) originally described by Altschul et al. 1990 (J Mol Biol 215: 403-10), such as the "Blast 2 sequences" algorithm described by Tatusova and Madden 1999 (FEMS Microbiol Lett 174: 247-250), for example using the published default settings or other suitable settings (such as, e.g., for the BLASTN algorithm: cost to open a gap = 5, cost to extend a gap = 2, penalty for a mismatch = -2, reward for a match = 1, gap x_dropoff = 50, expectation value = 10.0, word size = 28; or for the BLASTP algorithm: matrix = Blosum62, cost to open a gap = 11, cost to extend a gap = 1, expectation value = 10.0, word size = 3).

A variant of a protein, polypeptide or peptide may be a homologue (e.g., orthologue or paralogue) of said protein, polypeptide or peptide. As used herein, the term "homology" generally denotes structural similarity between two macromolecules, particularly between two proteins or polypeptides, from same or different taxons, wherein said similarity is due to shared ancestry.

Where the present specification refers to or encompasses fragments and/or variants of proteins, polypeptides or peptides, this preferably denotes variants and/or fragments which are "functional", i.e., which at least partly retain the biological activity or intended functionality of the respective proteins, polypeptides or peptides. Preferably, a functional fragment and/or variant may retain at least about 20%, e.g., at least 30%, or at least about 40%, or at least about 50%, e.g., at least 60%, more preferably at least about 70%, e.g., at least 80%, yet more preferably at least about 85%, still more preferably at least about 90%, and most preferably at least about 95% or even about 100% or higher of the intended biological activity or functionality compared to the corresponding protein, polypeptide or peptide.

Depending on factors that can be evaluated and decided on by a skilled person, such as inter alia the type of a biomarker (e.g., peptide, polypeptide, protein or nucleic acid), the type of a sample (e.g., whole blood, plasma, serum), the expected abundance of the biomarker in the sample, the type, robustness, sensitivity and/or specificity of the detection method used to detect the biomarker, etc., the biomarker may be measured directly in the sample, or the sample may be subjected to one or more processing steps aimed at achieving an adequate measurement of the biomarker.

By means of example, the sample may be subjected to one or more isolation or separation steps, aimed at whereby the biomarker is isolated from the sample or whereby a fraction of the sample is prepared which is enriched for the biomarker. For example, if the biomarker is a peptide, polypeptide, or protein, any known protein purification technique may be applied to the sample to isolate peptides, polypeptides, and proteins therefrom. Non-limiting examples of methods to purify peptides, polypeptides, or proteins, may include chromatography, preparative electrophoresis, centrifugation, precipitation, affinity purification, etc. For example, if the biomarker is a nucleic acid, such as RNA like mRNA, any known nucleic acid purification technique may be applied to the sample to isolate nucleic acids therefrom.

As used herein, the term "purified" with reference to markers, peptides, polypeptides, proteins or nucleic acids does not require absolute purity. Instead, it denotes that such markers, peptides, polypeptides, proteins or nucleic acids, are in a discrete environment in which their abundance (conveniently expressed in terms of mass or weight or concentration) relative to other analytes is greater than in the biological sample. A discrete environment denotes a single medium, such as for example a single solution, gel, precipitate, lyophilisate, etc. Purified proteins, polypeptides or peptides may be obtained by known methods including, for example, laboratory or recombinant synthesis, chromatography, preparative electrophoresis, centrifugation, precipitation, affinity purification, etc. Purified nucleic acids, such as RNA, like mRNA, may be obtained by known methods including, for example, mRNA purification columns.

Any existing, available or conventional separation, detection and quantification methods may be used herein to measure the presence or absence (e.g., readout being present vs. absent; or detectable amount vs. undetectable amount) and/or quantity (e.g., readout being an absolute or relative quantity, such as, for example, absolute or relative concentration) of markers, peptides, polypeptides, proteins or nucleic acids in samples. For example, such methods may include for peptides, polypeptides or proteins mass spectrometry analysis methods, biochemical assay methods, immunoassay methods, or chromatography methods, or combinations thereof. For example, such methods may include for nucleic acids like mRNA, (q)RT-PCR or digital PCR (e.g. droplet digital PCR).

In particular embodiments, the method comprises measuring at least IL-6 and SOCS3, and optionally S1PR1 and/or BCL3, in a biological sample from said subject. In particular embodiments, the method comprises measuring at least IL-6, SOCS3 and S1PR1, and optionally BCL3, in a biological sample from said subject. In particular embodiments, the method comprises measuring at least IL-6, SOCS3 and BCL3, and optionally S1PR1, in a biological sample from said subject. In particular embodiments, the method comprises measuring at least IL-6, SOCS3, S1PR1 and BCL3 in a biological sample from said subject.

In particular embodiments, the method further comprises measuring Janus kinase 1 (JAK1) and/or Signal transducer and activator of transcription 3 (STAT3) in said biological sample from the subject.

In particular embodiments, the method further comprises measuringPIM3, SLC29A1, MYC, CCR7, PIM2, OSM, TTLL12, DUSP7, and/or MAPKAPK3. Accordingly, in particular embodiments, the method comprises measuring at least IL-6 and SOCS3, and optionally one or more biomarkers selected from the group consisting of PIM3, SLC29A1, MYC, CCR7, PIM2, OSM, TTLL12, DUSP7, and/or MAPKAPK3.

In particular embodiments, not all biomarkers are measured at the same timepoint. For example, MYC may be measured at from 16 to 24 hours after the timepoint when the maintenance dose of said allergy immunotherapeutic is at least reached by the cumulative dose of said allergy immunotherapeutic, while one or more, such as at least two, preferably at least the first two, or more biomarkers selected from the group consisting of IL-6, SOCS3, S1PR1 and BCL3 are measured at the timepoint when the maintenance dose of said allergy immunotherapeutic is at least reached by the cumulative dose of said allergy immunotherapeutic. Accordingly, MYC may be measured at a later time point and may serve as a specificity validation biomarker for the other biomarkers IL-6, SOCS3, S1PR1 and/or BCL3. In particular embodiments, the method does not comprise measuring any biomarker other than IL-6 and SOCS3 in a biological sample from said subject. In particular embodiments, the method does not comprise measuring any biomarker other than IL-6, SOCS3, S1PR1 and BCL3 in a biological sample from said subject. In particular embodiments, the method does not comprise measuring any biomarker other than IL-6, SOCS3, S1PR1, BCL3, JAK1, STAT3, PIM3, SLC29A1, MYC, CCR7, PIM2, OSM, TTLL12, DUSP7 and MAPKAPK3 in a biological sample from said subject.

AIT typically comprises an up-dosing phase during which the subject is contacted with gradually increasing doses of an allergy immunotherapeutic until a maintenance dose of said allergy immunotherapeutic is reached followed by a maintenance phase during which the subject is contacted with said maintenance dose.

The term "maintenance dose" as used herein refers to the constant dosage which is envisaged to be used for a given subject during immunotherapy to ensure an effective immune response. For example, the maintenance dose (e.g. daily maintenance dose) may be from 50 to 300 µg or from 50 to 200 µg, such as about 100 µg of allergy immunotherapeutic. The person skilled in the art will understand that the maintenance dose is a predefined value representing the dose to be used during immunotherapy that is expected to ensure an effective immune response in a subject responsive to the AIT, as it is typically not known before initiating the AIT whether or not the subject will be responsive to the AIT (and hence whether an effective immune response will indeed be achieved). An appropriate maintenance dose of an allergy immunotherapeutic as taught herein may be determined by a qualified physician with due regard to the nature of the allergy immunotherapeutic, the disease condition and severity, and the age, size and condition of the patient.

In particular embodiments, the maintenance dose refers to a (total) daily maintenance dose. This means that the maintenance dose may be administered to the subject in one or multiple times (e.g. by one or multiple injections). For example, a maintenance dose of about 100 µg may be administered to the subject by two administrations of about 50 µg in one day.

The biomarker is preferably measured around the time when the cumulative dose administered to the subject during the up-dosing phase corresponds to the maintenance dose envisaged for said subject. Thus, for instance, if the maintenance dose is envisaged to be 100µg/day, the biomarker is preferably measured at a time point when the patient has received a cumulative dose of around 100µg. In particular embodiments, the biomarker(s) is/are measured at a time point in the period spanning from 3 hours before to 24 hours after the timepoint on which the cumulative dose of allergy immunotherapeutic during the up-dosing phase reaches the maintenance dose of said allergy immunotherapeutic.

In particular embodiments, the biomarker(s) is/are measured at a time point in the period spanning from 3 hours before (such as from 2 hours before, 1 hour before or 0.5 hour before) to 24 hours after, from 3 hours before to 20 hours after, from 3 hours before to 18 hours after, from 3 hours before to 16 hours after, from 3 hours before to 14 hours after, from 3 hours before to 12 hours after, from 3 hours before to 10 hours after, from 3 hours before to 8 hours after, from 3 hours before to 6 hours after, from 3 hours before to 4 hours after, from 3 hours before to 2 hours after, from 3 hours before to 1 hour after, the timepoint on which the cumulative dose of allergy immunotherapeutic during the up-dosing phase at least reaches the maintenance dose of said allergy immunotherapeutic.

In particular embodiments, the biomarker(s) is/are measured at the timepoint on which the cumulative dose of allergy immunotherapeutic during the up-dosing phase at least reaches 50%, 55%, 60%, 65%, 70%, 75%, preferably 80%, such as 85%, more preferably 90%, such as 95%, 96%, 97%, 98%, or 99%, most preferably 100% of the maintenance dose of said allergy immunotherapeutic, or at most 24 hours after the timepoint on which the cumulative dose of allergy immunotherapeutic during the up-dosing phase reaches the maintenance dose of said allergy immunotherapeutic.

In particular embodiments, the biomarker(s) is/are measured at the timepoint on which the cumulative dose of allergy immunotherapeutic during the up-dosing phase at least reaches the maintenance dose of said allergy immunotherapeutic or at most 24 hours, at most 18 hours, at most 20 hours, at most 18 hours, at most 16 hours, at most 14 hours, at most 12 hours, at most 10 hours, at most 8 hours, at most 6 hours, at most 4 hours, at most 2 hours, such as at most 1 hour, thereafter. In particular embodiments, the biomarker(s) is/are measured at the timepoint on which the cumulative dose of allergy immunotherapeutic during the up-dosing phase at least reaches the maintenance dose of said allergy immunotherapeutic.

The term "cumulative dose" as used herein refers to the sum of the subsequent individual doses of allergy immunotherapeutic being administered to the subject. For example, if the subject is being administered subsequent and gradually increasing doses of allergy immunotherapeutic of 0.003 µg, 0.006 µg, 0.015µg, 0.030 µg, 0.060 µg, 0.150 µg, 0.30 µg, 0.60 µg, 1.50 µg, 3.00 µg, 6.00 µg, 15.00 µg, 30.00 µg and 55.00 µg, the cumulative dose after these 14 individual doses is 111.66 µg.

In particular embodiments, the method comprises a step of selecting a maintenance dose prior to measuring the one or more, such as at least two, preferably at least the first two, biomarkers selected from the group consisting of IL-6, SOCS3, S1PR1 and BCL3 in the biological sample from the subject. Preferably this step is performed prior to subjecting the subject to said AlT.

In particular embodiments, the method comprises a step of selecting a dosage regime for said allergy immunotherapeutic, wherein said dosage regime comprises an up-dosing phase during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose. Preferably this step is performed prior to subjecting the subject to said AlT.

The person skilled in the art will understand that the cumulative dose of allergy immunotherapeutic during the up-dosing phase may reach the maintenance dose of allergy immunotherapeutic within one day or one week, but also within less than 5 hours, such as within 3 hours, such as described in Stock et al;, Safety and tolerability of venom immunotherapy: Evaluation of 581 rush- and ultra-rush induction protocols -safety of rush and ultra-rush venom immunotherapy, World Allergy Organization Journal, 2021, 14:100496.

In particular embodiments, the biomarker(s) is/are measured at a timepoint in the period spanning from 5 to 32 hours, from 5 to 24 hours, from 5 to 20 hours, from 5 to 16 hours, from 5 to 14 hours, from 5 to 12 hours, from 5 to 10 hours, from 5 to 9 hours, from 7 to 12 hours, from 7 to 10 hours, from 8 to 32 hours, from 8 to 30 hours, from 8 to 28 hours, from 8 to 26 hours, from 8 to 24 hours, from 8 to 20 hours, from 8 to 16 hours, from 8 to 14 hours, from 8 to 12 hours, from 8 to 10 hours, from 8 to 9 hours, from 8 to 12 hours, from 8 to 10 hours, preferably from 7 to 9 hours after the start of said AIT, such as insect-venom AIT, in said subject. The person skilled in the art will understand that the start of an AIT in said subject corresponds to the administration (e.g. by injection, preferably subcutaneous injection) of the first dose of an allergy immunotherapeutic in said subject.

In particular embodiments, the method comprises measuring the quantity or expression levels of at least one, preferably at least two, such as at least three or all four, biomarkers selected from the group consisting of consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic .

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, i.e., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g., weight per volume or mol per volume.

A relative quantity of a molecule or analyte in a sample may be advantageously expressed as an increase or decrease or as a fold-increase or fold-decrease relative to said another value, such as relative to a reference value or reference sample as taught herein. Performing a relative comparison between first and second parameters (e.g., first and second quantities) may but need not require first to determine the absolute values of said first and second parameters. For example, a measurement method can produce quantifiable readouts (such as, e.g., signal intensities) for said first and second parameters, wherein said readouts are a function of the value of said parameters, and wherein said readouts can be directly compared to produce a relative value for the first parameter vs. the second parameter, without the actual need first to convert the readouts to absolute values of the respective parameters.

The terms "quantity" and "expression level" of said at least one, preferably at least two, such as at least three or all four, biomarkers selected from the group consisting of IL-6, SOCS3, S1PR1 and BCL3, preferably at least IL-6 and SOCS3, are used interchangeably in this specification to refer to the absolute and/or relative quantification, concentration level or amount of any such product in a sample. In particular embodiments, the biomarker is a protein and the term "expression level" refers to the "protein expression level". In particular embodiments, the biomarker is mRNA and the term "expression level" refers to the "mRNA expression level".

Present inventors found that, in responders to (i.e. subjects responsive to) AIT, biomarkers IL-6, SOCS3, S1PR1 and/or BCL3 are significantly differentially expressed in a biological sample from a subject responding to an AIT, such as an insect-venom AIT, at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 24 hours, after the start of said insect-venom AIT in said subject) compared to a biological sample from the same subject before, at the start or at most 0.5 hour after the start of the AIT, such as insect-venom AIT, in said subject. Such differential expression was not observed in subjects not receiving said particular AIT (e.g. insect-venom AIT), such as pollen-allergy patients, who received injection AIT using pollen extract, or healthy subjects.

Accordingly, in particular embodiments, the methods as taught herein may comprise the step of comparing the quantity or expression levels of said at least one, preferably at least two, biomarkers selected from the group consisting of IL-6, SOCS3, S1PR1 and BCL3, preferably at least IL-6 and SOCS3, as measured with a reference, such as a reference sample. In particular embodiments, the reference is the quantity or expression levels of said at least one, preferably at least two, biomarkers selected from the group consisting of IL-6, SOCS3, S1PR1 and BCL3, preferably at least IL-6 and/or SOCS3, before, at the start or at most 0.5 hour after the start of the AIT, such as the insect-venom AIT, in said subject. In particular embodiments, the reference is a reference sample, wherein said reference sample is a sample from said subject before, at the start or at most 0.5 hour after the start of the AIT, such as the insect-venom AIT, in said subject.

In particular embodiments, the reference sample is a sample from said subject taken just before (e.g. at most 5 hours, at most 4 hours, at most 3 hours, at most 2 hours, at most 1 hour, at most 0.5 hours, before) the administration of the first dose of insect-venom allergy immunotherapeutic in said subject.

In particular embodiments, the method does not comprise comparing any biomarker other than IL-6 and SOCS3, any biomarker other than IL-6, SOCS3, S1PR1 and BCL3, or any biomarker other than IL-6, SOCS3, S1PR1, BCL3, JAK1, STAT3, PIM3, SLC29A1, MYC, CCR7, PIM2, OSM, TTLL12, DUSP7, and MAPKAPK3, in a biological sample from said subject.

In particular embodiments, the reference sample is a sample of a pollen allergy patient subjected to pollen AlT. In particular embodiments, the reference sample is a sample of a pollen allergy patient subjected to pollen AIT at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 hours to 15 weeks, such as from 14 days to 15 weeks, or from 6 to 12 weeks, after the start of said pollen AIT) in said subject.

In particular embodiments, said reference sample is a sample from a healthy subject. The healthy subject is typically a subject that has no known allergies. In particular embodiments, the healthy subject does not receive AlT.

In particular embodiments, if the biomarker is serological IL-6 protein, the reference sample may be a sample from a healthy subject.

As explained, the present methods, uses, or products may involve finding a difference or no difference between the quantity or expression level of said one or more (e.g. at least one, preferably at least two, such as at least three or all four) biomarkers selected from the group consisting of IL-6, SOCS3, S1PR1 and BCL3, preferably at least IL6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 7 to 9 hours or from 8 to 9 hours, after the start of said AIT) in said subject and the quantity or expression level of said one or more (e.g. at least one, preferably at least two, such as at least three or all four,) biomarkers selected from the group consisting of IL-6, SOCS3, S1PR1 and BCL3, preferably at least IL6 and SOCS3, in a reference.

A "difference" between a first value and a second value may generally encompass any direction (e.g., increase: first value > second value; or decrease: first value < second value) and any extent of alteration. In the present case, the first value may be the quantity or expression levels of the biomarker(s) at a timepoint in the period spanning from 3 hours before to 24 hours after the timepoint when the maintenance dose of said allergy immunotherapeutic is at least reached by the cumulative dose of said allergy immunotherapeutic and the second value may be the reference.

For example, a difference may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a difference may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a difference may refer to a statistically significant observed alteration. For example, a difference may refer to an observed alteration which falls outside of error margins of reference values in a given population (as expressed, for example, by standard deviation or standard error, or by a predetermined multiple thereof, e.g., ±1×SD or ±2×SD or ±3×SD, or ±1×SE or ±2×SE or ±3×SE). A difference may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a difference may be concluded if an observed alteration is beyond a given threshold or cut-off. Such threshold or cut-off may be selected as generally known in the art to provide for a chosen accuracy, sensitivity and/or specificity of the prediction methods, e.g., accuracy, sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal threshold or cut-off value of the quantity of a given biomarker for clinical use of the present diagnostic tests, based on acceptable global accuracy, sensitivity and/or specificity, or related performance measures which are well-known *per se,* such as positive predictive value (PPV), negative predictive value (NPV), positive likelihood ratio (LR+), negative likelihood ratio (LR-), Youden index, or similar.

For example, an optimal threshold or cut-off value may be selected for each individual biomarker as a local extremum of the receiver operating characteristic (ROC) curve, i.e. a point of local maximum distance to the diagonal line, as described in Robin X., PanelomiX: a threshold-based algorithm to create panels of biomarkers, 2013, Translational Proteomics, 1(1):57-64.

The person skilled in the art will understand that it is not relevant to give an exact threshold or cut-off value. A relevant threshold or cut-off value can be obtained by correlating the sensitivity and specificity and the sensitivity/specificity for any threshold or cut-off value.

It is to the diagnostic engineers to determine which level of positive predictive value/negative predictive value/sensitivity/specificity is desirable and how much loss in positive or negative predictive value is tolerable. The chosen threshold or cut-off level could be dependent on other diagnostic parameters used in combination with the present method by the diagnostic engineers.

The present methods, uses, or products may further involve attributing any finding of a difference or no difference between the quantity or expression level of said at least one, preferably at least two, such as at least three or all four, biomarkers selected from the group consisting of consisting of IL-6, SOCS3, S1PR1 and BCL3 measured in a biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 9 hours, after the start of an insect-venom AIT in said subject )and a given reference value or threshold to the responsiveness or unresponsiveness to the AIT (such as the insect-venom AIT). The reference value or threshold may be the quantity or expression level of said at least one, preferably at least two, such as at least three or all four, biomarkers selected from the group consisting of consisting of IL-6, SOCS3, S1PR1 and BCL3 measured in a reference sample.

In particular embodiments, the observation of a difference between the quantity or expression level of said at least one, preferably at least two, such as at least three or all four, biomarkers selected from the group consisting of consisting of IL-6, SOCS3, S1PR1 and BCL3 in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 9 hours, after the start of an insect-venom AIT in said subject) and a reference can lead to the conclusion that the responsiveness to the AIT (such as the insect-venom AIT) in said subject is different from that represented by said reference. Similarly, in particular embodiments, when no difference is found between the quantity or expression level of said at least one, preferably at least two, such as at least three or all four, biomarkers selected from the group consisting of consisting of IL-6, SOCS3, S1PR1 and BCL3 in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 9 hours, after the start of said insect-venom AIT in said subject) and a reference, the absence of such difference can lead to the conclusion that responsiveness to the AIT (such as the insect-venom AIT) in said subject is substantially the same as that represented by said reference.

Distinct references may represent the prediction of the responsiveness to an AIT (such as an insect-venom AIT) *vs.* the prediction of unresponsiveness to said AIT (such as said insect-venom AIT).

In particular embodiments, the reference as used in the methods as taught herein is determined from a biological sample from a subject or a group of healthy subjects.

In particular embodiments, the reference as used in the methods as taught herein is determined from a biological sample from a subject or a group of subjects known to be unresponsive to said AIT (such as said insect-venom AIT), such as one or more pollen-allergy patients. In particular embodiments, the pollen allergy patient is subjected to pollen AlT.

In particular embodiments, the reference as used in the methods as taught herein is determined from a biological sample from a subject or a group of subjects before, at the start or at most 0.5 hour after the start of the AIT, such as insect-venom AIT, in said subject. Preferably, the subject is the same subject as for who the responsiveness is being predicted. As described elsewhere herein, the person skilled in the art will understand that the start of an AIT, such as insect-venom AIT, in said subject corresponds to the administration (e.g. by injection, preferably subcutaneous injection) of the first dose of allergy immunotherapeutic (such as insect-venom allergy immunotherapeutic) in said subject.

The quantity or expression level of said at least one, preferably at least two, such as at least three or all four, biomarkers selected from the group consisting of IL-6, SOCS3, S1PR1 and BCL3, preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 7 to 9 hours or from 8 to 9 hours, after the start of said AIT in said subject) may be elevated compared to (i.e., relative to) the reference as described elsewhere herein. The so-elevated quantity or expression level may allow for the prediction of responsiveness of the subject to said AIT, such as said insect-venom AlT.

In particular embodiments, the method comprises the steps of
(a) measuring the quantity or expression levels of at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (e.g. at a timepoint in the period spanning from 5 to 24 hours, preferably from 7 to 9 hours or from 8 to 9 hours, after the start of said AIT, such as insect-venom AIT, in said subject);
(b) comparing the quantity or expression levels of said at least one, preferably at least two, such as at least three or all four, biomarkers selected from the group consisting of IL-6, SOCS3, S1PR1 and BCL3, more preferably at least IL-6 and SOCS3, as measured in (a) with a reference, preferably wherein said reference is the quantity or expression levels of said at least one, preferably at least two, biomarkers selected from the group consisting of IL-6, SOCS3, S1PR1 and BCL3, more preferably at least IL-6 and SOCS3, before, at the start or at most 0.5 hour after the start of the AIT, such as the insect-venom AIT, in said subject;
(c) finding a difference or no difference of the quantity or expression levels of said at least one, preferably at least two, biomarkers selected from the group consisting of IL-6, SOCS3, S1PR1 and BCL3, more preferably at least IL-6 and SOCS3, as measured in (a) from said reference; and
(d) attributing said finding of difference or no difference to a particular prediction of responsiveness of the subject to said AIT, such as said insect-venom AlT.

In particular embodiments, an increase (i.e. an elevation) in the quantity or expression levels of at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as insect-venom AIT) compared to the reference is indicative of responsiveness of the subject to said AIT, such as said insect-venom AlT.

In particular embodiments, such as if the biomarker is serological IL-6 protein, comparison of the quantity of IL-6 as measured with a reference may not be required to predict the responsiveness of the subject to the AIT, especially when considered in combination with one or more other biomarker(s) as described herein. In particular embodiments, such as if the biomarker is serological IL-6 protein, a quantity of from 1 to 8 pg, from 1.5 to 8 pg, from 1.6 to 8 pg, from 1.6 to 7 pg, from 1.6 to 6 pg, from 1.6 to 5 pg, from 1.6 to 4 pg, or from 1.7 to 8 pg of IL-6 protein per ml of whole blood is indicative of responsiveness of the subject to said AlT. In particular embodiments, such as if the biomarker is serological IL-6 protein, a quantity of less than 1.7 pg, less than 1.6 pg or less than 1.5 pg of IL-6 protein per ml of whole blood is indicative of non-responsiveness of the subject to said AlT.

Present inventors have also shown that the natural circadian rhythm is of importance during sampling for clinical investigations related to dynamic immune responses. Accordingly, in particular embodiments, the biological sample from said subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT) and said reference sample were taken at approximately the same time of day. In particular embodiments, such as if the reference is taken from the same subject as the subject for who the responsiveness to AIT, such as insect-venom AIT, is being predicted, said reference is measured in a second biological sample from said subject, wherein the first biological sample and said second biological sample are taken at approximately the same time of day. For example, if the biological sample from said subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT) was taken in the morning, such as at about 8 am, said reference sample is preferably also taken in the morning, such at about 8 am.

A further aspect provides a kit, in particular a kit for predicting the response (or sensitivity or susceptibility) of a subject having an allergy, such as an insect-venom allergy to an AIT, such as an insect-venom AIT, the kit comprising means specifically adapted for measuring the quantity or expression levels of at least one, preferably at least two, such as at least three or all four, biomarkers selected from the group consisting of IL-6, SOCS3, S1PR1 and BCL3, more preferably at least IL-6 and SOCS3, in a biological sample from a subject. In particular embodiments, the kit further comprises means specifically adapted for measuring the quantity or expression levels of PIM3, SLC29A1, MYC, CCR7, PIM2, OSM, TTLL12, DUSP7, and/or MAPKAPK3.

The terms "kit of parts" and "kit" as used throughout this specification refer to a product containing components necessary for carrying out the specified methods, packed so as to allow their transport and storage. Materials suitable for packing the components comprised in a kit include crystal, plastic (e.g., polyethylene, polypropylene, polycarbonate), bottles, flasks, vials, ampules, paper, envelopes, or other types of containers, carriers or supports. Where a kit comprises a plurality of components, at least a subset of the components (e.g., two or more of the plurality of components) or all of the components may be physically separated, e.g., comprised in or on separate containers, carriers or supports. The components comprised in a kit may be sufficient or may not be sufficient for carrying out the specified methods, such that external reagents or substances may not be necessary or may be necessary for performing the methods, respectively. Typically, kits are employed in conjunction with standard laboratory equipment, such as liquid handling equipment, environment (e.g., temperature) controlling equipment, analytical instruments, etc. In addition to the recited set of isolated oligonucleotides as taught herein, optionally provided on arrays or microarrays, the present kits may also include some or all of solvents, buffers (such as for example but without limitation histidine-buffers, citrate-buffers, succinate-buffers, acetate-buffers, phosphate-buffers, formate buffers, benzoate buffers, TRIS (Tris(hydroxymethyl)-aminomethan) buffers or maleate buffers, or mixtures thereof), enzymes (such as for example but without limitation thermostable DNA polymerase), detectable labels, detection reagents, and control formulations (positive and/or negative), useful in the specified methods. Typically, the kits may also include instructions for use thereof, such as on a printed insert or on a computer readable medium. The terms may be used interchangeably with the term "article of manufacture", which broadly encompasses any man-made tangible structural product, when used in the present context.

In particular embodiments, said means is a binding agent specifically binding to said biomarker (e.g. protein) or to RNA encoding said biomarker (e.g. protein). Such binding agents are known in the art and include primers, probes and/or antibodies, but are not limited thereto.

In particular embodiments, said kit comprises at least one, preferably at least two, such as at least three or all four, of the following binding agents:
- a binding agent specifically binding to the IL-6 polypeptide or the RNA encoding the IL-6 polypeptide;
- a binding agent specifically binding to RNA encoding the SOCS3 polypeptide;
- a binding agent specifically binding to RNA encoding the S1PR1 polypeptide;
- a binding agent specifically binding to RNA encoding the BCL3 polypeptide;
- a binding agent specifically binding to RNA encoding the PIM3 polypeptide;
- a binding agent specifically binding to RNA encoding the SLC29A1 polypeptide;
- a binding agent specifically binding to RNA encoding the MYC polypeptide;
- a binding agent specifically binding to RNA encoding the CCR7 polypeptide;
- a binding agent specifically binding to RNA encoding the PIM2 polypeptide;
- a binding agent specifically binding to RNA encoding the OSM polypeptide;
- a binding agent specifically binding to RNA encoding the TTLL12 polypeptide;
- a binding agent specifically binding to RNA encoding the DUSP7 polypeptide; and/or
- a binding agent specifically binding to RNA encoding the MAPKAPK3 polypeptide.

Preferably, said kit comprises at least a binding agent specifically binding to the IL-6 polypeptide or the RNA encoding the IL-6 polypeptide and a binding agents specifically binding to RNA encoding the SOCS3 polypeptide. Optionally, the kit further comprises a binding agent specifically binding to RNA encoding the S1PR1 polypeptide and/or a binding agent specifically binding to RNA encoding the BCL3 polypeptide.

In particular embodiments, the kit is an *in situ* hybridization assay that combines branched-DNA technology with flow cytometry, such as the Prime Flow RNA assay kit (Thermo Fisher Scientific; catalog number 88-18005-210).

In particular embodiments, the kit does not comprise any means specifically adapted for measuring the quantity or expression levels of a biomarker other than IL-6, SOCS3, S1PR1, BCL3, JAK1, STAT3, PIM3, SLC29A1, MYC, CCR7, PIM2, OSM, TTLL12, DUSP7, and MAPKAPK3.

A further aspect provides the use of the kit as taught herein for predicting the response (or sensitivity or susceptibility) of a subject having an allergy, such as an insect-venom allergy, to an AIT, such as an insect-venom AIT, based on the detection of at least one, preferably at least two, such as at least three or all four, biomarkers selected from the group consisting of IL-6, SOCS3, S1PR1 and BCL3, more preferably at least IL-6 and SOCS3, in a biological sample of a subject.

A further aspect provides the use of the kit as taught herein in a method as taught herein, such as the *in vitro* method as taught herein for predicting responsiveness (or sensitivity or susceptibility) to an AIT, such as an insect-venom AIT, in a subject having an allergy, such as an insect-venom allergy, the method as taught herein for indicating an AIT, such as an insect-venom AIT, as a suitable treatment for an allergy, such as an insect-venom allergy, in a subject or the method as taught herein for predicting an AIT, such as an insect-venom AIT, outcome in a subject having an allergy, such as an insect-venom allergy.

A further aspect provides an allergy immunotherapeutic or allergen immunotherapeutic agent, such as an insect-venom allergy immunotherapeutic or insect-venom allergen immunotherapeutic agent, for use in a method of treating an allergy, such as an insect-venom allergy, (or a method of preventing an allergic reaction) in a subject, wherein said method comprises predicting responsiveness (or sensitivity or susceptibility) of said subject to said immunotherapeutic using a method as taught herein and continuing administering said immunotherapeutic to said subject if the subject is predicted to be responsive to said immunotherapeutic.

In particular embodiments, the allergy immunotherapeutic is an aqueous composition, more particularly an aqueous pharmaceutical composition, optionally comprising one or more pharmaceutically acceptable carriers.

In particular embodiments, the allergy immunotherapeutic comprises one or more allergens or immunogenic fragment(s) thereof. In particular embodiments, the allergy is an allergy against an animal and the allergen is an allergen from said animal. In particular embodiments, the animal is a domestic animal such as a cat or a dog. In further particular embodiments, the animal is an insect or a reptile. In further embodiments, the animal is a rodent.

In particular embodiments, the allergy immunotherapeutic further comprises one or more adjuvants, as described elsewhere herein.

In particular embodiments, the allergy immunotherapeutic is an insect-venom allergy immunotherapeutic. In particular embodiments, the insect-venom allergy immunotherapeutic comprises one or more insect-venom allergens (or immunogenic fragments thereof). Insect-venom allergens are known in the art and can be found on the WHO/IUIS Allergen Nomenclature homepage http://www.allergen.org/. In particular embodiments, the insect-venom allergy immunotherapeutic may comprise a honeybee (*Apis mellifera*) allergen, such as, but not limited to, Api m 1, Api m 2, Api m 3, Api m 4, Api m 5, Api m 6, Api m 7, Api m 8, Api m 9, Api m 10, Api m 11.0101, Api m 11.0201, or Api m 12, preferably Api m1 or Api m5. In particular embodiments, the insect-venom allergy immunotherapeutic may comprise a Vespula (*Vespula vulgaris*) allergen, such as, but not limited to, Ves v 1, Ves v 2.0101, Ves v 2.0201, Ves v 3, Ves v 5 or Ves v 6, preferably Ves v 1 or Ves v 5. In particular embodiments, the insect-venom allergy immunotherapeutic may comprise a Polistes (*Polistes dominula*) allergen, such as, but not limited to, Pol d 1, Pol d 3, Pol d 4 or Pol d 5. In particular embodiments, the insect-venom allergy immunotherapeutic may comprise one or more ant (e.g. fire ant) allergens or immunogenic fragments thereof. In particular embodiments, the insect-venom allergy immunotherapeutic may comprise one or more allergens (or immunogenic fragment(s) thereof) of the European/American hornet (e.g., *Vespa crabro*) or the Asian hornet *Vespa velutina* e.g. nigrithorax, or *Megachile sculpturalis,* or any invasive hymenoptera species (e.g., *Afranthidium (Immanthidium) repetitum*)*.* In particular embodiments, the allergy immunotherapeutic is a cat-allergy AlT. In particular embodiments, the cat allergy immunotherapeutic comprises one or more cat allergens (or immunogenic fragments thereof). Non-limiting examples of cat allergens include Fel d 1.

In particular embodiments, the allergy immunotherapeutic, such as the insect-venom allergy immunotherapeutic, is administered to the subject as part of a conventional, a cluster, a rush or an ultra-rush scheme for an allergy immunotherapeutic, such as an insect-venom allergen immunotherapy. AIT, such as insect-venom AIT, typically consists of build-up (i.e. up-dosing) and maintenance phases to ensure a sustained effect. More particularly, said AIT may comprise an up-dosing phase during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose. The time to reach the maintenance dose depends on the scheme used-namely, conventional, rush, ultra-rush, or cluster. Compared to conventional protocol, ultra-rush protocol has several advantageous such as shorter build-up time, fewer hospital/doctor's visits, optimum patient compliance, and less labour and time waste.

In particular embodiments, the allergy immunotherapeutic, such as the insect-venom allergy immunotherapeutic, is administered to the subject via a series of subcutaneous injections.

A non-limiting example of an ultra-rush insect-venom AIT comprises a build-up phase comprising the administration of 14 injections of an insect-venom allergy immunotherapeutic in 8 hours followed by subsequent injections at day 7 and 14. Afterwards, the injections are performed monthly for a minimum of 3 up to 5 years. A non-limiting example of conventional protocol consists of 17 injections (one weekly) with cumulative dose of 332.14 µg (0.02 µg, 0.04 µg, 0.08 µg, 0.2 µg, 0.4 µg, 0.6 µg, 0.8 µg, 2 µg, 4 µg, 6 µg, 8 µg, 10 µg, 20 µg, 40 µg, 60 µg, 80 µg and 100 µg).

A further aspect provides a method of treating an allergy, such as an insect-venom allergy, in a subject in need of such a treatment, comprising continuing administering a therapeutically effective amount of an allergy immunotherapeutic or allergen immunotherapeutic agent, such as an insect-venom allergy immunotherapeutic or insect-venom allergen immunotherapeutic agent, to the subject, wherein the subject has been selected as having an allergy, such as an insect-venom allergy, characterised by an increased quantity or expression levels of at least two biomarkers selected from the group consisting of IL-6, SOCS3, S1PR1 and BCL3, preferably at least IL-6 and SOCS3,in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT) compared to a reference, as described elsewhere herein.

The person skilled in the art will understand that in the methods as taught herein, the AIT is started to subsequently predict the responsiveness (or sensitivity or susceptibility) to said AlT. Said AIT is continued upon performing the methods as taught herein. Or in other words, the subject is being administered one or more subsequent doses of an allergen immunotherapeutic agent. However, once a subject has been predicted to be non-responsive to said AIT, the AIT is discontinued, the dosage regime for said allergy immunotherapeutic is changed, or an alternative therapeutic product (e.g. an alternative allergy immunotherapeutic) is administered..

A related aspect provides a method of treating an allergy, such as an insect-venom allergy, in a subject in need of such a treatment, the method comprising the steps of
(a) measuring the quantity or expression levels of at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 9 hours, after the start of said AIT, such as said insect-venom AIT, in said subject);
(b) comparing the quantity or expression levels of said at least one, preferably at least two, such as at least three or all four, biomarkers selected from the group consisting of IL-6, SOCS3, S1PR1 and BCL3, more preferably at least IL-6 and SOCS3, as measured in (a) with a reference, preferably wherein said reference sample is the quantity or expression levels of said at least one, preferably at least two, such as at least three or all four, biomarkers selected from the group consisting of IL-6, SOCS3, S1PR1 and BCL3 more preferably at least IL-6 and SOCS3, before, at the start or at most 0.5 hour after the start of the AIT, such as the insect-venom AIT, in said subject;
(c) finding a difference or no difference of the quantity or expression levels of said at least one, preferably at least two, such as at least three or all four, biomarkers selected from the group consisting of IL-6, SOCS3, S1PR1 and BCL3, more preferably at least IL-6 and SOCS3, as measured in (a) from said reference; and
(d) (i) if the quantity or expression levels of said at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT) is increased compared to the reference, continuing administering a therapeutically effective amount of an allergy immunotherapeutic, such as an insect-venom allergy immunotherapeutic, to the subject;
(d) (ii) if the quantity or expression levels of said at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT) is not increased compared to the reference, discontinuing administering a therapeutically effective amount of an allergy immunotherapeutic, such as an insect-venom allergy immunotherapeutic, to the subject, changing the dosage regime for said allergy immunotherapeutic, or administering an alternative therapeutic product (e.g. an alternative allergy immunotherapeutic) to the subject.

A related aspect provides the use of an allergy immunotherapeutic, such as an insect-venom allergy immunotherapeutic, for the manufacture of a medicament for the treatment of an allergy, such as an insect-venom allergy, in a subject, wherein the subject has been selected as having an allergy, such as an insect-venom allergy, characterized by an increased quantity or expression levels of at least two biomarkers selected from the group consisting of IL-6, SOCS3, S1PR1 and BCL3, preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT)compared to a reference, as described elsewhere herein.

A related aspect provides the use of an allergy immunotherapeutic, such as an insect-venom allergy immunotherapeutic, for the manufacture of a medicament for the treatment of an allergy, such as an insect-venom allergy, in a subject, wherein the subject has been selected for treatment by a method comprising the steps of
(a) measuring the quantity or expression levels of at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours, preferably from 8 to 9 hours, after the start of said AIT, such as said insect-venom AIT in said subject);
(b) comparing the quantity or expression levels of said at least one, preferably at least two, such as at least three or all four, biomarkers selected from the group consisting of IL-6, SOCS3, S1PR1 and BCL3, more preferably at least IL-6 and SCOS3,as measured in (a) with a reference, preferably wherein said reference is the quantity or expression levels of said at least one, preferably at least two, such as at least three or all four, biomarkers selected from the group consisting of IL-6, SOCS3, S1PR1 and BCL3, more preferably at least IL-6 and SOCS3, before, at the start or at most 0.5 hour after the start of the insect-venom AIT in said subject;
(c) finding a difference or no difference of the quantity or expression levels of said at least one, preferably at least two, such as at least three or all four, biomarkers selected from the group consisting of IL-6, SOCS3, S1PR1 and BCL3, more preferably at least IL-6 and SOCS3, as measured in (a) from said reference; and
(d) (i) if the quantity or expression levels of said at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT, is increased compared to the reference, continuing administering a therapeutically effective amount of an allergy immunotherapeutic, such as an insect-venom allergy immunotherapeutic to the subject;
(d) (ii) if the quantity or expression levels of said at least one, preferably at least two, such as at least three or all four, biomarkers selected from the group consisting of IL-6, SOCS3, S1PR1 and BCL3, more preferably at least IL-6 and SOCS3, in the biological sample from the subject at a timepoint in the period spanning from 3 hours before to 24 hours after the timepoint when the maintenance dose of said allergy immunotherapeutic is at least reached by the cumulative dose of said allergy immunotherapeutic (such as at a timepoint in the period spanning from 5 to 24 hours after the start of said AIT, such as said insect-venom AIT, is not increased compared to the reference, discontinuing administering a therapeutically effective amount of an allergy immunotherapeutic, such as an insect-venom allergy immunotherapeutic to the subject, changing the dosage regime for said allergy immunotherapeutic, or administering an alternative therapeutic product (e.g. an alternative allergy immunotherapeutic) to the subject.

As used herein, a phrase such as "a subject in need of treatment" includes subjects that would benefit from treatment of a given condition, particularly an allergy. Such subjects may include, without limitation, those that have been diagnosed with said condition, those prone to develop said condition and/or those in who said condition is to be prevented.

The terms "treat" or "treatment" encompass both the therapeutic treatment of an already developed disease or condition, such as the therapy of an already developed allergy, as well as prophylactic or preventive measures, wherein the aim is to prevent or lessen the chances of incidence of an undesired affliction, such as to prevent occurrence, development and progression of an allergy. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. The term "therapeutically effective amount" as used herein, refers to an amount of active compound or pharmaceutical agent that elicits the biological or medicinal response in a subject that is being sought by a researcher, veterinarian, medical doctor or other clinician, which may include inter alia alleviation of the symptoms of the disease or condition being treated. Methods are known in the art for determining therapeutically and prophylactically effective doses for the immunotherapeutic or pharmaceutical formulation as taught herein.

The products and methods as taught herein allow to administer a therapeutically effective amount of an allergy immunotherapeutic, such as an insect-venom allergy immunotherapeutic, as taught herein in subjects having an allergy, such as an insect-venom allergy, which will benefit from such treatment. The term "therapeutically effective amount" as used herein, refers to an amount of active compound or pharmaceutical agent that elicits the biological or medicinal response in a subject that is being sought by a surgeon, researcher, veterinarian, medical doctor or other clinician, which may include inter alia alleviation of the symptoms of the disease or condition being treated. Methods are known in the art for determining therapeutically effective doses of an allergy immunotherapeutic, such as an insect-venom allergy immunotherapeutic, as taught herein.

The term "therapeutically effective dose" as used herein refers to an amount of an allergy immunotherapeutic as taught herein, that when administered brings about a positive therapeutic response with respect to treatment of a patient having an allergy.

Appropriate therapeutically effective doses and dosing schemes of an allergy immunotherapeutic as taught herein may be determined by a qualified physician with due regard to the nature of the allergy immunotherapeutic, the disease condition and severity, and the age, size and condition of the patient.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and scope of the appended claims.

The herein disclosed aspects and embodiments of the invention are further supported by the following non-limiting examples.

### Example 1. IL-6, SOCS3, S1PR1 and BCL3 as early-phase biomarkers for predicting the responsiveness to an insect-venom AIT

### Materials and methods

### 1.1 Cohort design.

The study was approved by the Luxembourg National Research Ethics Committee (CNER) (SYS-T-Act; 201509/11) and registered at ClinicalTrials.gov (NCT02931955). Informed consent was obtained from each participant prior to sampling and personal data collecting, which were in full compliance with the Luxembourg National Commission for Data Protection (CNPD, before the implementation of GDPR in Europe).

The study participants with moderate-to-severe allergic rhinitis (pollen allergy patients, PAP, n=16) and insect sting reactions (venom allergy patients, VAP, n=18) eligible for AIT in accordance with the current international guidelines were recruited at a tertiary center (Service National d'Immuno-Allergology of Centre Hospitalier de Luxembourg) between August 2016 and February 2018. The healthy controls (HC, n=10) were recruited at the Clinical and Epidemiological Investigation Center of the Luxembourg Institute of Health (CIEC-LIH) between Jan 2018 and Apr 2018. The study was designed as a real-world, prospective, exploratory, data-driven trial for identification of novel potential biomarkers and molecular switches in the early time window of AIT in the outpatient clinical setting. The inclusion criteria were the confirmed clinical diagnosis of allergy (skin prick test positivity and elevated slgE titers to plant pollen or insect venom allergens, respectively), clinical indication for launch of AIT for the patients and absence of known allergies for healthy volunteers. The exclusion criteria were age less than 18 years, pregnancy and lactation; overt asthma or chronic obstructive pulmonary disease, concomitant autoimmune disorders, chronic diseases in exacerbation or not adequately controlled, history of hematological malignancies or solid tumors; treatment with systemic steroids, immunomodulatory agents or biologics, acute and exacerbation of chronic infections, traumas and surgeries in six months prior to the study enrollment. No restrictions were introduced regarding the number of sensitizing allergens, duration of the allergic disease, the time elapsed since the last field sting and treatment with mono- versus polyvalent allergen immunotherapy products. In VAP, an ultra-rush AIT protocol was administered to reach the maintenance dose within 8 hours with 14 injections (Figure 3A and Table 3). For PAP, a conventional up-dosing AIT was applied to reach the maintenance dose within 6 weeks with a weekly injection scheme. Therefore, although the exact sampling periods are different between the two groups of patients, present inventors' sampling schemes allowed them to cover comparable periods (essentially the build-up phase of AIT) until the beginning of the maintenance stage for both types of AlT.

**Table 3**

| Injection, No | Allergen concentration, ug/mL | Dose, ml | Injected dose, ug |
|---|---|---|---|
| 1 | 0,01 | 0.30 | 0.003 |
| 2 | 0,01 | 0.60 | 0.006 |
| 3 | 0,1 | 0.15 | 0.015 |
| 4 | 0,1 | 0.30 | 0.030 |
| 5 | 0,1 | 0.60 | 0.060 |
| 6 | 1 | 0.15 | 0.150 |
| 7 | 1 | 0.30 | 0.30 |
| 8 | 1 | 0.60 | 0.60 |
| 9 | 10 | 0.15 | 1.50 |
| 10 | 10 | 0.30 | 3.00 |
| 11 | 10 | 0.60 | 6.00 |
| 12 | 100 | 0.15 | 15.00 |
| 13 | 100 | 0.30 | 30.00 |
| 14 | 100 | 0.55 | 55.00 |
| 15 | 100 | 0.50 | 50.00 |
| 16 | 100 | 0.50 | 50.00 |
| 17 | 100 | 0.50 | 50.00 |
| 18 | 100 | 0.50 | 50.00 |

| | | | |
|---|---|---|---|
| *Day 1: injections 1-14 (updosing); Day 7: injections 15 and 16 (maintenance); Day 14: injections 17 and 18 (maintenance); updosing phase: 0h-8h and maintenance phase: starting from 8h (**Fig. 3**); cumulative dose after 14 injections is 111.66 µg; daily maintenance dose: 100 µg* | | | |

While the age of the PAP was comparable to that of HC (median, ~37 and ~35 years, respectively), VAP were also middle-aged, but slightly older (median, ~49 years). While both patient groups included male and female participants at almost 1:1 ratio, the HC group was predominantly female (nine of 10). Five of 16 PAP were sensitized to major birch pollen allergen (Bet v 1), two of 16 exhibited elevated slgE and clinical reactivity to timothy grass pollen allergens (Phl p 1 & Phl p 5) and eight were allergic to the pollen of both species. One PAP was timothy grass pollen-allergic, but the slgE results were not available. Most VAP (14 of 18) presented with a history of systemic reactions to wasp (yellow jacket; Vespula spp.) stings, being sensitized to Ves v 1 (one of 18), Ves v 5 (eight of 18) or both allergens (five of 18). Three of four bee VAP showed elevated slgE levels to both Api m 1 and Api m 5, another VAP being mono-sensitized to Api m 1. Two VAP are hobby beekeepers.

Biological samples from patients and HC were collected at 8 AM and 4 PM (corresponding to baseline and 8h in VAP and HC groups) by trained study nurses, transported within maximum two hours to the bio-specimen laboratory of the Integrated Biobank of Luxembourg (IBBL), where they were either used freshly in the experiments on the same day, or cryopreserved in the IBBL biorepository for further analysis.

### 1.2 Measurement of circulating cytokine levels by the MSD assay.

Ten cytokines or chemokines (IFN-y, IL-1β, IL-2, IL-4, IL-6, IL-8, IL-10, IL-12p70, IL-13 and TNF-a) were measured in plasma samples (isolated from EDTA tubes) from patients and healthy controls at all time points using a multiplex assay [The V-Plex Proinflammatory Panel 1 (hu) assay from MSD, kit catalog K15049D-1]. The samples were undiluted and measured in duplicates. The assay was performed according to the manufacturer's instructions. Data were recorded and analyzed on a MESO QuickPlex SQ 120 instrument (software version LSR_4_0_12).

### 1.3 Th2-cell-type-specific RNA-seq analysis.

### Sorting and sequencing Th2 cells

Following the PBMC isolation from fresh blood, CD4+ T cells were first enriched with human CD4 microbeads (130-045-101, Miltenyl Biotec) following the manufacture's recommendations while the remaining cells were immediately cryopreserved as described above. The CD4+ T cells were then immediately shipped to our sorting flow-cytometry facility at 4°C. The CD4+ T cells were first stained in Brilliant Stain Buffer (563794, BD) using all the abs with specified dilution factors listed in **Table 2.** Incubate for 30 min at 4°C in the dark, briefly vortex once after 15 min. Wash the cells twice: centrifuge at 350g, 10 min, 4°C. Re-suspend the cells at a concentration of 10 million per ml. Proceed to aseptic sorting 20 PSI, with 100 um nozzle and Purity mode in a BD Aria III sorter (for the gating strategy, please refer to the paragraph relating to the FACS sorting strategy below). After sorting, cells were centrifuged at 350g, 10 min, 4°C, pelleted and lysated in 700 ul of the Qiazol buffer (Qiagen). Transfer the samples into 1.5 ml Eppendorf tubes and immediately freeze at -80°C until RNA extraction. RNA was extracted using RNeasy Micro kit (Qiagen) following the manufacture's recommendations. RNA-seq were performed at the EMBL Genomics Core Facility (Heidelberg, Germany) using pair-end 75bp by NexSeq 500 with ^{~} 50 Million reads/sample.

**Table 2. The list of flow cytometry abs used to sort Th2 cells from each participant at each time point.**

| **Target** | **Alternativ e name** | **Fluorochro me** | **Clon e** | **Used volume ul/10⁶ cells** | **Catalogue#, Manufactur er** |
|---|---|---|---|---|---|
| **L/D** | Near IR for 633 or 635 excitation | | | 1 | L34976, Thermo Fisher Scientific |
| **CD4** | | BUV805 | SK3 | 4 | 564910, BD |
| **CD183** | CXCR 3 | Bv421 | 1C6/ CXCR 3 | 3 | 562558, BD |
| **CD45R A** | | Pacific Blue | HI10 0 | 2 | 304123, Biolegend |
| **CD3** | | BV510 | HIT3 a | 3 | 564713, BD |
| **CD8** | | Bv650 | RPA-T8 | 1 | 301042, Biolegend |
| **CD127** | IL-7R | Bv711 | A019 D5 | 4 | 351328, Biolegend |
| **CD25** | IL-2RA | BB5 15 | 2A3 | 3 | 564467, BD |
| **CD45R O** | | PE-CF594 | UCHL 1 | 2 | 562299, BD |
| **CD196** | CCR6 | PE-Cy7 | 11A9 | 4 | 560620, BD |
| **CD194** | CCR4 | APC | L291 H4 | 2 | 359408, Biolegend |

### FACS-sorting strategy to define living CD4 Th2 singlets (CD3⁺CD4⁺CD8⁻ non-Treg then CD45RO⁺CD45RA⁻CD183/CXCR3⁻CD196/CCR6⁻CD194/CCR4⁺).

The gating strategy was performed as follows. The lymphocyte population was determined by their characteristic of forward (FSC-A) and side scatter (SSC-A) properties and the doublets (using FSC-A vs FSC-H) were excluded. After the identification of live cells using live-dead staining markers and CD3 T-cells, the CD8⁻CD4⁺ cells were then selected. Then CD25^{low}CD127⁺ (known as non-Treg cells) were selected. Then CD45RO⁺CD45RA⁻ memory CD4 T cells were selected. Then CD183⁻CD196⁻ cells were selected. Finally CD194⁺ cells were selected as the targeted Th2 cells and sorted out for expression analysis.

### Th2-cell-specific RNA-seq pre-processing and quality control

Quality control of the raw sequencing data was conducted using the FastQC software (v0.11.7-Java-1.8.0_162) (https://www.bioinformatics.babraham.ac.uk/projects/fastqc/). Reads were mapped to the reference human genome 38 (http://hgdownload.soe.ucsc.edu/goldenPath/hg38/bigZips/) and summarized to genes using the R Bioconductor Rsubread package (v1.32.4). In addition to those failed with RNA extraction due to technical issues, one sample was also excluded for further analysis because CD127 Ab was not added before sorting.

### Analysis of potential confounding factors

Clinical and demographic factors that are significantly different between groups can have a confounding effect on subsequent analyses if no correction for these factors is applied. The following statistical tests were conducted to identify potential confounding factors. For categorical variables, a Fisher's exact test was conducted. For normally distributed numerical variables having equal variances, one-way ANOVA was applied. In case of a normally distributed variable with unequal variances between the groups, Welch one-way test was conducted. For non-normally distributed values, the Kruskal-Walls rank sum test was applied. Homogeneity of variances was determined using Levene's test, while normality was tested using quantile-quantile (Q-Q) plots.

### Filtering, normalization and voom transformation

Genes that consistently had zero or very low counts were first removed using the Bioconductor edgeR package (v3.24.3). Trimmed Mean of M-values (TMM) normalization was performed on the filtered data with the edgeR package. The normalized data were voom-transformed to enable differential expression analysis with the Bioconductor limma package (v3.38.3).

### Quality control on voom-transformed data

Principal component analysis (PCA) was performed on the voom-transformed data, and a score plot was presented where each batch was represented by a different colored symbol. In case of no batch effect, the batches do not cluster in the PCA plot. Furthermore, a principal coordinates analysis (PCoA) was conducted with the R Stats package and the score plot was inspected to check for potential outliers.

### Differential expression and enrichment analysis

Differentially-expressed genes (DEGs) between allergic patients and controls, and between AIT-treated samples and baseline were determined by applying an empirical Bayes moderated t-test on the voom-transformed data, implemented in the R Bioconductor limma package (v3.38.3). Nominal p-values were corrected for multiple hypothesis testing by computing false discovery rates (FDR) according to the Benjamini-Hochberg procedure and using an FDR significance threshold of 0.05. Entrez gene IDs were converted to gene symbols with the R Bioconductor org.Hs.eg.db package (v3.7.0). DEGs were visualized in heatmaps and volcano plots using the R Bioconductor ComplexHeatmap (v1.20.0) and R ggplot2 (v3.2.0) packages, respectively.

Enriched differentially-regulated pathways and GO (Gene Ontology) biological processes were determined by over-representation analysis in ConsensusPathDB (release 34, http://cpdb.molgen.mpg.de/) using the list of all measured genes as background list. Pathways were selected from Reactome, KEGG, Wikipathways, Biocarta and PID. Pathways and GO biological processes with Benjamini-Hochberg corrected FDR ≤ 0.05 were considered significant.

### Results

A temporary but uniform enhancement of IL-6 signaling in Th2 cells of VAP immediately following AIT launch. As Th2 cells are vital effectors of allergic responses that need to be stringently controlled through AIT, present inventors comprehensively analyzed the underlying molecular changes in Th2 cells of allergic patients and performed a genome-scale RNA-seq analysis in sorted purified Th2 cells (**data not shown and Table 2**). Cell-type specific RNA-seq analysis, showing a certain degree of granularity between bulk and single-cell RNA-seq methods, can reveal highly-accurate transcriptomic profiles for a given purified cell type, especially for a rare subpopulation, e.g., Th2 cells. Similar to present inventors'CyTOF results (**data not shown**), the frequency of sorted *ex-vivo* Th2 among living CD4 T cells was indeed much higher in VAP vs. HC (**Figure 1A**) and the frequency of sorted Th2 cells was highly correlated with the frequency of Th2 cells in the CyTOF analysis (**Figure 1B**)**.** Present inventors' RNA-seq analysis did not show any culprit-allergen bias (**data not shown**)**.** All the marker genes specific for Th2 but not Th1, Th17 and Tregs were highly expressed among 10 randomly-selected RNA-seq samples (**data not shown**)**.** Interestingly, present inventors' RNA data also exhibited a diurnal distribution pattern of several key circadian regulatory genes in sorted Th2 cells. The expression level of the key circadian rhythm genes, such as *NR1D1* (also known as *REV-ERBα*) was uniformly increased in Th2 cells at 8h vs. baseline among almost all VAP following AIT (p-value=4.0E-4, **Figure 2A**)**.** Among HC, another circadian regulatory gene, *PER3,* changed at 8h vs. baseline (p-value=0.0429, **Figure 2B**). Fluctuation in transcript levels of key circadian genes within Th2 cells and in frequency of circulating pDC (**data not shown**) together demonstrated the importance of considering natural circadian rhythm during sampling for clinical investigations related to dynamic immune responses.

Unexpectedly, the number of differentially-expressed genes (DEGs) immediately peaked in VAP at 8h after AIT onset, followed by a decline to a very-low level thereafter (**Figure 2C**). We then assessed whether those DEGs (**Figure 1C**) are significantly enriched in any molecular pathways or processes at 8h vs. baseline. Surprisingly, present inventors found the IL-6 signaling pathway was ranked on top (**Figure 1D**). Present inventors further asked which specific genes were affected in VAP at 8h following AlT. Through the unbiased volcano analysis, present inventors found three genes, namely *SOCS3, S1PR1* and *BCL3,* were substantially upregulated, thus being displayed as "outliers" (**Figure 1E**) in the upper right corner of the volcano plot. Suppressor of cytokine signaling 3 (SOCS3) is a central regulatory component of the IL-6 signaling pathway. Patients with genetic defects in IL-6 signaling, in particular those with homozygous mutations in IL-6Ra mainly present with primary atopy such as elevated IgE, eczema and eosinophilia. Present inventors' observation showed a very-early-stage temporarily, but uniformly increased *SOCS3* transcript response among VAP but not HC (**Figure 1F**), which therefore might contribute to the long-term high-curative outcomes of venom AlT. In line with the notion of enhanced IL-6 signaling after AIT launch in VAP, mRNA levels of *JAK1* and *STAT3* were also significantly upregulated at 8h vs. baseline in Th2 cells of VAP following AIT start (Figure 2D-E).

Present inventors' unbiased analysis identified *S1PR1* (sphingosine-1-phosphate receptor 1) as another significantly-upregulated gene in sorted Th2 cells at 8h vs. baseline (**Figure 1E****, G**). S1PR1 plays a critical role in regulating lymphocyte trafficking between blood and lymphoid organs. Although inhibiting S1PR1 slows egress of lymphocytes from lymph nodes and reduces the number of circulating lymphocytes, no obvious increase of Th2 cells was observed at 8h vs. baseline in VAP **(data not shown).** This most likely due to the compensatory upregulation of the chemokine receptor *CCR7* **(****Figure 2F****),** which supports the back migration of lymphocytes from blood to lymphoid organs .

Present inventors' transcriptome analysis also pinpointed a substantial increase (^{~} fourfold, **Figure 1H**) in mRNA levels of *BCL3,* a member of the non-canonical NFKB pathway, in Th2 cells among VAP at 8h vs. 0h. IL-6 induces BCL3 expression, like in in murine global CD4 T cells, also in human Th2 cells in a STAT3 (**Figure 2E**)-dependent manner. Concurrently, the kinase IKKα that critically activates the non-canonical NFKB pathway was also enhanced in PBMC depleted of CD4 T cells at 8h vs. baseline in VAP immediately following AIT launch **(data not shown).** In line with the notion of several temporarily-activated immune subsets as indicated by various layers of our multi-omics analysis, a T-cell survival- and homeostasis-regulatory gene, *SLC29A1* (Equilibrative Nucleoside Transporter 3, *ENT3)* , was also located in the upper right segment of the volcano plot (**Figure 1E****,** **Figure 2G**)**.** Present inventors further made use of our genome-scale *ex-vivo* time-series RNA-seq dataset to investigate whether any other pathways were enhanced during the AIT process by slightly reducing the fold-change thresholds while only confining to those genes showing a continuous dynamic pattern (**Figure 2H**)**.** Following this additional analysis, the detected 29 significantly-increased genes were significantly enriched in the pathways regulating mitotic cell cycle, inhibiting apoptosis, regulating MAPK signaling or JAK-STAT signaling (Figure 2H-M)**.** Several M-phase regulatory and apoptosis inhibitory genes, such as *PIM2* and *PIM3* were already upregulated in Th2 cells at 8h vs. baseline (**Figure 2J**)**.** Concordantly, the increase in kinase activity levels of PIM2 and PIM3 was also revealed in the unbiased kinome analysis (**data not shown**)**,** although in non-CD4 immune cell types. The other genes regulating MAPK and JAK-STAT signaling, such as *MYC, MAPKAPK3, DUSP7* and OSM [another IL-6 family cytokine) ] showed upregulated transcripts in Th2 cells one day after AIT launch (**Figure 2J****,** **2L**)**.** Another MAPK signaling molecule, fibroblast growth factor 18 (*FGF18*)*,* was increased in Th2 cells one week post AIT launch (**Figure 2M**)**.** Together, present inventors' unbiased Th2-specific transcriptomic analysis pinpointed several critical components in IL-6 signaling and the related pathways within 8-24h immediately following AIT launch as the molecular switches towards long-term immunological-tolerance reinstallation in VAP.

Having discovered the enhanced IL-6 signaling pathway in Th2 cells on the transcript level, present inventors' next asked whether serological IL-6 levels are also changed. Highly interestingly, among all the 10 analyzed Th1 (TNF-a, IFN-y, IL-12), Th2 (IL-4, IL-13) and other inflammation-related cytokines (**Figure 1I**), IL-6 was the only one showing a pattern of uniform and temporary upregulation immediately following AIT treatment among VAP (**Figure 1I****, J**)**.** Although the average increase was almost five- or four-fold at 8h or 24h relative to baseline, respectively, the absolute IL-6 concentration still remained in the near homeostatic non-inflammatory range (**Figure 1J**). Notably, the ROC (receiver operating characteristic) analysis showed that the non-inflammatory absolute IL-6 levels alone, especially at 8h (**Figure 4A****)** and 24h post AIT launch (**Figure 4B**), presented an outstanding diagnostic value [the area under curve (AUC) up to 0.9941] to distinguish VAP and HC. Since the main producers of IL-6 are APC and endothelial cells, APC-derived IL-6 plays a critical role in restricting Th2 differentiation and several types of APC were more abundant among VAP vs. HC (**data not shown**), present inventors' investigated a potential correlation between serological IL-6 levels and specific APC subsets or total APC. IL-6 levels were positively correlated with the frequency of total APC only in HC but not in PAP and VAP at baseline (**Figure 1K**)**.** In line with this notion, circulating IL-6 levels were highly correlated with the frequency of intermediate monocytes (iMono, CD3⁻CD56⁻CD19⁻CD20⁻HLA-DR⁺CD14^{dim}CD16^{dim}), classical monocytes (cMono) and total APC among HC, but not in VAP or PAP (**data not shown**), when samples from different time points were grouped together. Interestingly, the increased frequency of total APC at D7 in VAP relative to baseline was significantly correlated with the augmented IL-6 levels at 24h post AIT (**Figure 1L**)**.** These results indicate that the existing equilibrium between IL-6 and total APC or APC subsets in HC was disturbed in both groups of allergic patients. Importantly, present inventors'data show that AIT can restore this shift early on in the first days after AIT launch in VAP.

In contrast to the fast response in VAP, PAP displayed a substantial increase in the number of DEGs in Th2 cells only after the completion of the up-dosing phase, i.e., at 12w following AIT launch (**Figure 2C**)**,** which is also consistent with the slow dynamic evolution as suggested by our CyTOF analysis in PAP (**data not shown**)**.** The top-ranked genes with increased expression (p<=5e-4, fold change>=1.4) were significantly enriched in the pathways regulating mitochondrial functions and tRNA processing (**Figure 2N**)**,** indicating enhanced metabolic and translational activities, all preparing for cell activation and proliferation. The differential response dynamics on the molecular levels represented one of the key differences between VAP and PAP during the early phase of AIT, which may play an important role for regulating long-term AIT outcomes.

### Example 2. Increased IL-6 levels can also be used as early-phase biomarkers for predicting the responsiveness to a cat-allergy AIT.

The allergic mice were sensitized to Fel d 1 with Fel d 1 Al(OH)₃ and were later challenged with Fel d 1. The AIT mice group were primed with Fel d 1 Al(OH)₃ and received Fel d 1 CpG-adjuvanted AIT followed by Fel d 1 challenges. Our results show that IL-6 levels in various tissues such as sera, spleen and peritoneal cavity were increased at 24 hour following the start of AIT in mice vs allergic mice without AlT. These results indicate that increased circulating IL-6 levels can be also used as early-phase biomarker to predict the responsiveness to a cat-allergy AlT.

## Claims

1. An *in vitro* method for predicting responsiveness to an allergen immunotherapy (AIT) in a subject having an allergy, wherein said AIT comprises an up-dosing phase during which the subject is contacted with gradually increasing doses of an allergy immunotherapeutic until the cumulative dose of said allergy immunotherapeutic at least reaches a maintenance dose of said allergy immunotherapeutic followed by a maintenance phase during which the subject is contacted with said maintenance dose, the method comprising
measuring at least interleukin-6 (IL-6) and Suppressor Of Cytokine Signaling 3 (SOCS3) in a biological sample from said subject at a timepoint in the period spanning from 3 hours before to 24 hours after the timepoint when the maintenance dose of said allergy immunotherapeutic is at least reached by the cumulative dose of said allergy immunotherapeutic.

2. The method according to claim 1, further comprising measuring one or more biomarkers selected from the group consisting of Sphingosine-1-phosphate receptor 1 (S1PR1), B-cell lymphoma 3 (BCL3), Janus kinase 1 (JAK1) and Signal transducer and activator of transcription 3 (STAT3), preferably S1PR1 and/or BCL3, in said biological sample from the subject.

3. The method according to claim 1 or 2, wherein the biological sample is a whole blood sample.

4. The method according to any one of claims 1 to 3, wherein circulating IL-6 is measured in a whole blood sample, preferably in a serum sample or a plasma sample.

5. The method according to any one of claims 1 to 4, wherein SOCS3 and optionally one or more biomarkers selected from the group consisting of S1PR1, BCL3, JAK1 and STAT3 are measured in CD4 T helper (Tₕ) 2 cells.

6. The method according to any one of claims 1 to 5, wherein at least IL-6 and SOCS3, and optionally S1PR1 and/or BCL3 are measured at most 2 hours after the timepoint when the maintenance dose of said allergy immunotherapeutic is at least reached by the cumulative dose of said allergy immunotherapeutic.

7. The method according to any one of claims 1 to 6, comprising the steps of
(a) measuring the quantity or expression levels of at least IL-6 and SOCS3 in the biological sample from the subject at a timepoint in the period spanning from 3 hours before to 24 hours after the timepoint when the maintenance dose of said allergy immunotherapeutic is at least reached by the cumulative dose of said allergy immunotherapeutic;
(b) comparing the quantity or expression levels of at least IL-6 and SOCS3 as measured in (a) with a reference;
(c) finding a difference or no difference of the quantity or expression levels of at least IL-6 and SOCS3 as measured in (a) from said reference; and
(d) attributing said finding of difference or no difference to a particular prediction of responsiveness of the subject to said AlT.

8. The method according to claim 7, wherein said reference is the quantity or expression levels of at least IL-6 and SOCS3 before, at the start or at most 0.5 hour after the start of the AIT in said subject.

9. The method according to claim 8, wherein an increase in the quantity or expression levels of at least IL-6 and SOCS3 in the biological sample from the subject at a timepoint in the period spanning from 3 hours before to 24 hours after the timepoint when the maintenance dose of said allergy immunotherapeutic is at least reached by the cumulative dose of said allergy immunotherapeutic, compared to the reference is indicative of responsiveness of the subject to AlT.

10. The method according to any one of claims 1 to 9, wherein the subject is a human subject.

11. A kit, in particular a kit for predicting the response of a subject having an allergy to an AIT, the kit comprising means specifically adapted for measuring the quantity or expression levels of at least IL-6 and SOCS3, and optionally S1PR1 and/or BCL3, in a biological sample from a subject.

12. The kit according to claim 11, wherein said means comprises a binding agent specifically binding to IL-6 or to RNA encoding IL-6 and a binding agent specifically binding to SOCS3 or to RNA encoding SOCS3, and optionally a binding agent specifically binding to S1PR1 or to RNA encoding S1PR1 and/or a binding agent specifically binding to BCL3 or to RNA encoding BCL3.

13. Use of the kit according to claim 11 or 12 for predicting the response of a subject having an allergy to an AIT based on the detection of at least IL-6 and SOCS3 and optionally S1PR1 and/or BCL3 in a biological sample of a subject.

14. An allergy immunotherapeutic for use in a method of treating an allergy in a subject, wherein the method comprises predicting responsiveness of said subject to said allergy immunotherapeutic using a method according to any one of claims 1 to 10 and continuing administering said allergy immunotherapeutic to said subject if the subject is predicted to be responsive to said allergy immunotherapeutic.
